# EUROPEAN PATENT APPLICATION

(11) **EP 4 789 711 A1**
(43) Date of publication of application: **12.08.2026**
(21) Application number: 26157202.8
(22) Date of filing: 09.02.2026
(51) Int. Cl.: A61M 25/10, A61M 29/02

(54) **A BALLOON CATHETER SYSTEM**

(30) Priority: 10.02.2025 DK PA202530089
(71) Applicant: Venteus ApS, 2100 Copenhagen Ø (DK)
(72) Inventor: Gad, Jens, 2100 Copenhagen Ø (DK); Møller, Martin Nue, 2100 Copenhagen Ø (DK)
(74) Representative: Plougmann Vingtoft a/s

(57) **Abstract**

A balloon dilation system suitable for balloon dilation of an anatomic passageway, such as a Eustachian Tube and/or a Sinus passageway of an individual. The balloon dilation system (100) includes a balloon catheter (101) with a catheter shaft (103) having a proximal shaft portion (105), a distal shaft portion (107) and a shaft lumen (108); an inflatable balloon (102) in fluid connection with the shaft lumen and located at the distal shaft portion and a lumen port (111) at the proximal shaft portion and in fluid connection with the shaft lumen. The balloon dilation system includes an inflation arrangement having a syringe with a barrel and a syringe port in fluid connection with a barrel volume. The inflation arrangement has a grasping portion (126) engaged with, connected to or forming part of one of the plunger (120) and the barrel (118) and a thruster (127) engaged with, connected to or forming part of another one of the plunger and the barrel, wherein the balloon catheter and the inflation arrangement are coupled to or are integrated with each other in a rigid connection portion and wherein the grasping portion is located distally to the thruster and/or wherein the grasping portion is located with a shorter distance to a proximal balloon end than the thruster.

## Description

### TECHNICAL FIELD

The present invention relates to a medical balloon dilation system suitable for balloon dilation of an anatomic passageway of a person. The invention also relates to a method for balloon dilation of an anatomic passageway in the head of a person.

### BACKGROUND ART

Balloon dilation of an anatomic passageway of a person e.g., a Sinus passageways and/or a Eustachian tube of a person is a common procedure for treatment for example of chronic sinusitis and/or Eustachian Tube Dysfunction (ETD). EDT is characterized by the inability of the Eustachian tube to ventilate the middle ear. The Eustachian tube is a relatively narrow passage, e.g. 3-4 mm in diameter that connects the middle ear to the nasopharynx (upper throat and back of the nose). The Eustachian tube varies in size between individuals, and in addition, the Eustachian tube is placed deep inside the nose and may have an angled trajectory (about 30° in adults and more horizontal in children, which may make dilation thereof complex and painful to the treated person.

Patients with EDT often report multiple symptoms, such as a plugged feeling in the ears, ears feeling like they are filled with water, tinnitus, or ringing in the ear, muffled hearing or partial hearing loss, ticking or popping sounds, pain, and tenderness around the ear, a tickling or tingling sensation and trouble with balance. Additionally, ETD can lead to other more severe middle ear diseases. The potential patient population is huge, with prevalence of ETD being reported as high as 4.6% among the background population, which makes this a widespread disease by definition. Barometric related problems, such as when flying or diving exists in as many as 10% of cases. The socioeconomic burden of this disease should not be underestimated. Studies have reported that medical care visits associated with ETD exceed 4 million per annum in the US alone.

A challenge related to known equipment for such balloon dilation is that the balloon catheters applied may be either complex and/or difficult to use for a physician, and even for an experienced physician, the handling of the balloon catheter may cause difficulties.

Several attempts to improve the balloon catheters for performing such dilation problems have been performed, and in recent years, improved devices for performing balloon dilation of an anatomic passageway of a person have been provided.

WO 2024/008263 discloses a device for performing balloon dilation of an anatomic passageway of a person which has been improved with respect to handability and wherein the device may be operated with a single hand of the physician.

### DISCLOSURE OF INVENTION

However, there is still a need for new balloon dilation systems, which enable the physician to perform the dilation with a high or even an improved control.

An objective of the present invention is to provide a balloon dilation system, which ensures the physician to have high control of the balloon dilation system during the dilation procedure of an anatomic passageway, such as an Eustachian tube of a person and/or such as the Sinus passageways, preferably to provide that the dilation procedure may be performed relatively fast and with minimal discomfort to the person.

In an embodiment it is an objective to provide a balloon dilation system, which is relatively simple and fast to prepare for performing a dilation procedure of an anatomic passageway of a person,

In an embodiment it is an objective to provide a balloon dilation system, which ensures a correct and effective dilation of e.g. the Eustachian tube of a person, and with low risk or even practically no risk of damaging the patient.

In an embodiment it is an objective to provide a balloon dilation system, which may be relatively simple to adapt for performing a dilation procedure of an anatomic passageway of an individual person to thereby ensure that the dilation procedure may be performed in a relatively simple way and with a desired dilation effect and with minimal discomfort to the person.

In an embodiment it is an objective to provide a method of balloon dilation of an anatomic passageway of a person which is relatively fast and cost effective and which causes a minimum of pain to the person.

These and other objectives have been solved by the invention or embodiments thereof as defined in the claims and/or as described herein below.

It has been found that the invention or embodiments thereof have a number of additional advantages, which will be clear to the skilled person from the following description.

The inventor of the present invention has realized that by providing the balloon dilation system to comprise a balloon catheter and an inflation arrangement which are integrated with or coupled to each other in a rigid connection portion, the balloon dilation system may be operated with a very high control by the physician and simultaneously, the physician may in a simple way ensure an inflation of the balloon of the catheter with a high accuracy to ensure a correct dilation of the anatomic passageway, such as the Eustachian tube. In addition, by providing that the thruster and the grasping portion are arranged with an optimized architecture, it has been found that the balloon dilation system is relatively simple and uncomplicated to handle by a physician, which has shown to make the dilation procedure relatively fast and cost effective while simultaneously ensuring that potential pain that the person may be exposed to is reduced to a minimum.

The term "substantially" should herein be taken to mean that ordinary product variances and tolerances are comprised.

The term "length section L" means herein a length section from a clamp location to a force applying location when using the single sided clamp method and when using the three-point-bending test method the length section L means the distance between two supports.

It should be emphasized that the term "comprises/comprising" when used herein is to be interpreted as an open term, i.e. it should be taken to specify the presence of specifically stated feature(s), such as element(s), unit(s), integer(s), step(s) component(s) and combination(s) thereof, but does not preclude the presence or addition of one or more other stated features.

Throughout the description or claims, the singular encompasses the plural unless otherwise specified or required by the context.

The "an embodiment" should be interpreted to include examples of the invention comprising the feature(s) of the mentioned embodiment.

The term "about" is generally used to include what is within measurement uncertainties. When used in ranges the term "about" should herein be taken to mean that what is within measurement uncertainties is included in the range.

The term "substantially" should herein be taken to mean that ordinary product variances and tolerances are comprised.

All features of the invention and embodiments of the invention as described herein, including ranges and preferred ranges, may be combined in various ways within the scope of the invention, unless there are specific reasons not to combine such features.

The term "rigid" is herein used to mean that a rigid element is stiff at least at standard conditions of 1 atm. and 23 °C.

The terms "person", "patient" and "individual" are used interchangeably.

The terms "physician", "operator" and "user" are used interchangeably.

Unless other is specified, any properties, ranges of properties and/or determinations, such as stiffness properties and/or determinations are to be determined at standard conditions of 23°C and 1 atmosphere (1.01325 Bar) unless otherwise specified.

Reference made to "some embodiments" or "an embodiment" means that a particular feature(s), structure(s), or characteristic(s) described in connection with such embodiment(s) is included in at least one embodiment of the subject matter disclosed. Thus, the appearance of the phrases "in some embodiments" or "in an embodiment" in various places throughout the specification is not necessarily referring to the same embodiment(s). Further, the skilled person will understand that particular features, structures, or characteristics may be combined in any suitable manner within the scope of the invention as defined by the claims.

The balloon dilation system of the invention is advantageously suitable for balloon dilation of an anatomic passageway, such as described above. In an embodiment the balloon dilation system is suitable for dilation of a Eustachian tube of a person. In an embodiment, the balloon dilation system is suitable for dilation of one or all of the Sinus passageways. In an embodiment, the balloon dilation system is suitable for both of the Eustachian tube of a person and also some or all of the Sinus passageways.

The balloon dilation system of the invention comprises a balloon catheter and an inflation arrangement.

The balloon catheter comprises
a catheter shaft having a shaft length, a shaft axis, a proximal shaft portion, a distal shaft portion and a shaft lumen,
an inflatable balloon located at the distal shaft portion, the inflatable balloon having a proximal balloon end and a distal balloon end and the inflatable balloon being in fluid connection with the shaft lumen, and
a lumen port at the proximal shaft portion in fluid connection with the shaft lumen.

The inflation arrangement comprises
a syringe comprising a barrel having a barrel volume, a syringe axis, and a plunger with a plunger rod and a plunger head,
a syringe port in fluid connection with the barrel volume,
a grasping portion engaged with, connected to or forming part of one of the plunger and the barrel,
a thruster engaged with, connected to or forming part of another one of the plunger and the barrel.

The barrel volume of the inflation arrangement is in fluid connection with the shaft lumen of the balloon dilation system to provide that fluid may be fed to the balloon for expanding the balloon once the balloon is located in the anatomic passageway to be dilated.

The balloon dilation system comprises a rigid connection portion and the balloon catheter and the inflation arrangement are coupled to or are integrated with each other in the rigid connection portion, preferably such that a motion of the inflation arrangement may be directly correlated with a corresponding motion of the balloon catheter.

Advantageously, the inflation arrangement has a distal end defined by the syringe port and wherein the grasping portion is located distally to the thruster and/or the grasping portion is located with a first distance d1 to the proximal balloon end, and the thruster may be located with a second distance d2 to the proximal balloon end and wherein the second distance d2 is larger than the first distance d1.

Thereby the thruster may be located proximally to the grasping portion, which ensures that the balloon dilation system may be relatively simple and uncomplicated to handle by a physician, which may ensure that the dilation procedure becomes relatively fast and cost effective.

In an embodiment, the grasping portion and the thruster are displaceable relative to each other, such as linearly displaceable relative to each other, e.g. displaceable along a displacement line parallel to the syringe axis.

The grasping portion may conveniently be located with the first distance D1 to the proximal balloon wherein the first distance D1 is constant, while the thruster is displaceable relative to the grasping portion.

The first distance D1 and the second distance D2 is determined as the maximal distance, i.e. the first distance D1 is determined from the proximal balloon end and to a point of the grasping portion located furthest from the proximal balloon end and the second distance is determined from the proximal balloon end and to a point of the thruster located furthest from the proximal balloon end.

In an embodiment, the rigid connection portion is at least linear rigid, meaning that the rigid connection portion ensures that all linear motions of the inflation arrangement are directly transferred to the balloon catheter, while rotational motions of the inflation arrangement and/or the balloon catheter may not be fully or partly transferred, such that the inflation arrangement and the balloon catheter for example may be rotated, such as up to 180°, such as up to 90°, such as up to 45° relative to each other.

Advantageously, the rigid connection portion is fully rigid meaning that the rigid connection portion is rigid also against rotations such that both rotational and linear motions of the inflation arrangement are directly transferred to the balloon catheter.

In an embodiment, the balloon dilation system may be configured for being switched between a first mode wherein the rigid connection portion is linear rigid and a second mode wherein the rigid connection portion is fully rigid.

Thereby the surgeon may adjust the balloon catheter and the inflation arrangement rotationally relative to each other when the balloon dilation system is in the first mode - e.g. prior to inserting the balloon catheter into the anatomic passageway to be dilated, whereafter the surgeon may switch the balloon dilation system to the second mode for safe and accurate guiding of at least the distal portion of the balloon catheter into the anatomic passageway. The switching between the first and the second mode may e.g. be provided by a displaceable slider, which in the second mode prevents and/or blocks relative rotation between the balloon catheter and the inflation arrangement and in the first mode allows relative rotation between the balloon catheter and the inflation arrangement.

Preferably, the proximal shaft portion extends at least 50 mm from the rigid connection portion. The optimal length of the proximal shaft portion depends on the size and shape of the anatomic passageway to be dilated and may differ from person to person.

In an embodiment, the proximal shaft portion extends at least 50 mm from the rigid connection portion, and the proximal shaft portion has a flexural stiffness of at least 1000 Nmm² determined in a length section L extending from the rigid connection portion to 50 mm or more from the rigid connection portion.

Advantageously, the length section L extending from the rigid connection portion to 50 mm or more from the rigid connection portion has a flexural stiffness of at least 5000 Nmm², such as at least 10000 Nmm², such as at least 25000 Nmm², such as at least 50000 Nmm², such as at least 75000 Nmm², such as at least 80000 Nmm², for example determined by the three-point-bending test method and/or by the single sided clamp method as described below and/or in according to ASTM D790 and/or ASTM D6272.

The flexural stiffness may be determined from E (Youngs modulus) and I (Second moment of area) according to the formula E*I = Flexural stiffness.

The skilled person will know how to determine the flexural stiffness in a length section L of an elongate item such as of the catheter shaft, e.g. of the proximal shaft portion.

In an embodiment, a stiffness parameter is determined using a single sided clamp method comprising clamping an elongate item to be tested e.g. the proximal shaft portion, at a clamp location, applying a force F at a force applying location at a distance from the from the clamp location corresponding to the length section L and transverse to the elongate item and determine the stiffness in the form of
- a deflection 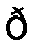 of the proximal shaft portion at the force applying location by applying a preselected force F, or
- a required force F for providing a preselected deflection 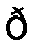 of the proximal shaft portion at the force applying location, or
- a value k representing force-to-deflection ratio, determined by applying a force F and determining the deflection and determining k according to the F/ 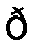 ratio.

The flexural stiffness (E*I) can be determined from the above k stiffness parameters using the formula $E * I = F * {L}^{3} / \left(3*ð\right) .$

Where the rigid connection portion has a flexural stiffness equal to or larger than such as at least 50% larger than the stiffness of the proximal shaft portion, the rigid connection portion may be considered to provide the clamping such that the clamp location is adjacent a proximal shaft end of the proximal shaft portion.

Determined e.g. by a three-point-bending test method or by the single sided clamp method.

In an embodiment, a stiffness parameter is determined using a three-point bending test, preferably comprising placing the item to be tested, such as the proximal shaft portion or a part thereof in horizontal orientation onto two supports located with a distance corresponding to the length section L, applying a force F transverse to the item at a center location along the length section L of the item, to determine the maximal deflection of the item and determining the flexural stiffness El according to the formula $EI = \frac{F ∗ {L}^{3}}{48 ∗ ð}$ wherein El is the flexural stiffness, F is the applied force, L is the distance between the two supports and 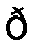 is the deflection.

In an embodiment, the proximal shaft portion extends at least 70 mm from the rigid connection portion and wherein the proximal shaft portion has a flexural strength determined in a length section L extending from the rigid connection portion to 70 mm or more from the rigid connection portion, wherein the flexural stiffness is at least 1000 Nmm². Preferably the length section L extending from the rigid connection portion to 70 mm or more from the rigid connection portion has a flexural stiffness of at least 5000 Nmm², such as at least 10000 Nmm², such as at least 25000 Nmm², such as at least 50000 Nmm², such as at least 75000 Nmm², such as at least 80000 Nmm². The flexural stiffness may be determined as described above.

In an embodiment, where the proximal shaft portion is determined in a length section L extending from the rigid connection and in the entire proximal shaft portion, the proximal shaft portion has a flexural stiffness of at least 1000 Nmm², preferably the length section L extending from the rigid connection and in the entire proximal shaft portion has a flexural stiffness of at least 5000 Nmm², such as at least 10000 Nmm², such as at least 25000 Nmm², such as at least 50000 Nmm², such as at least 75000 Nmm², such as at least 80000 Nmm², e.g. determined as described above.

In an embodiment, the proximal shaft portion has a stiffness determined using the single sided clamp method, wherein the clamp location is adjacent a proximal shaft end of the proximal shaft portion and the force applying location is adjacent the proximal shaft portion distal end and wherein the proximal shaft portion has a stiffness sufficiently high to prevent the proximal shaft portion distal end from deflecting more than 20 mm when a force of 1N is applied to the proximal shaft portion at a location adjacent to the proximal shaft portion distal end in a direction transverse to the shaft axis, preferably the proximal shaft portion distal end has a deflection not exceeding 18 mm, such as not exceeding 16 mm, such as not exceeding 14 mm when a force of 1N is applied to the proximal shaft portion at a location adjacent to the proximal shaft portion distal end in a direction transverse to the shaft axis.

Advantageously, the proximal shaft portion has a force-to-deflection ratio k determined using the single sided clamp method, wherein the clamp location is adjacent a proximal shaft end of the proximal shaft portion and the force applying location is adjacent the proximal shaft portion distal end and wherein the force-to-deflection ratio k is at least 0.1 N/mm, such as at least 0.2 N/mm, such as, at least 0.3 N/mm, such as, at least 0.4 N/mm, such as at least 0.5 N/mm, such as at least 0.6 N/mm, such as at least 0.7 N/mm, such as at least 0.8 N/mm.

It has been found that by ensuring that the proximal shaft portion has a relatively high stiffness the coordination between motion of the inflation arrangement and the distal shaft portion, including the inflatable balloon provides the physician with a high perception of the location and motions of the distal shaft portion and especially about the location of the inflatable balloon, which may simplify the procedure and make it easier and faster for the physician to positioning the inflatable balloon in the correct position in the anatomic passageway.

Whereas in prior art balloon dilation systems it has been a general belief that the balloon shaft needed to be flexible to avoid damaging and/or causing excessive pain to the person under treatment, the inventor of the present invention has realized that due the increased perception of the physician caused by a relatively high stiffness of the proximal shaft portion, the physician may guide the distal shaft portion with a very high accuracy, which thereby may cause less pain to the person in particular also caused by the fact that the procedure may be performed faster and with less futile attempts.

The rigid connection portion may conveniently interconnect the balloon catheter and the inflation arrangement. Advantageously, the rigid connection portion has a flexural stiffness larger than or identical to the flexural stiffness of at least a length section L extending from the rigid connection portion to 50 mm or more from the rigid connection portion. Preferably, the rigid connection portion has a flexural stiffness which is at least 105%, such as at least 110%, such as at least 120% of the flexural stiffness of at least the length section L extending from the rigid connection portion to 50 mm or more from the rigid connection portion. Thereby, the balloon dilation system may become very stable and safe for use.

In an embodiment, the proximal shaft portion comprises or consists of a length portion of the catheter shaft comprising an annular metallic reinforcement layer. This may conveniently be in the form of a shaft hypotube, an externally located steel tube and/or an annular reinforcement. As explained in more details below, the annular metallic reinforcement layer may have several purposes and effects.

Advantageously, the balloon dilation system comprises a fluid connection between the syringe port and the lumen port, wherein the fluid coupling beneficially comprises a valve arrangement for closing and opening the fluid connection.

In an embodiment, the fluid connection between the barrel volume and the shaft lumen is provided by a fluid coupling optionally directly between the syringe port and the lumen port. The fluid coupling between the barrel volume and the shaft lumen may constitute or form part of the rigid connection portion, wherein the fluid coupling optionally comprises an annular reinforcement, such as a metallic reinforcement ring.

In an embodiment, the fluid connection between the barrel volume and the shaft lumen is provided via a tube section, such as a flexible tube section.

As explained, the balloon dilation system may in an embodiment be in a first mode wherein the rigid connection portion is a linear rigid connection portion allowing rotational motions of the balloon catheter and the inflation arrangement relative to each other.

In an embodiment, the rigid connection portion is in a fully rigid connection portion wherein all motions of the entire inflation arrangement are translated to the balloon catheter.

The rigid connection portion may comprise a coupling, such as a mechanical coupling. The mechanical coupling may comprise the fluid coupling. In an embodiment, the mechanical coupling is a separable mechanical coupling.

The mechanical coupling may in an embodiment comprise a threaded connection between the balloon catheter and the inflation arrangement.

In an embodiment, the mechanical coupling comprises a snap-lock between the balloon catheter and the inflation arrangement. The snap-lock may be irreversible or releasable.

Thereby, the operator may, in a relative way and without switching the inflation arrangement, switch from one balloon catheter to another balloon catheter having a different catheter shaft while using the same inflation arrangement. Since the anatomic passageway differs from one individual to another, it is quite normal that the operator needs to try two or more balloon catheters before finding the optimal size and most suitable balloon catheter.

The balloon catheter and the inflation arrangement may for example be mechanically coupled by a click-on locking mechanism to ensure a desired rigidity of the rigid connection portion.

In an embodiment, the rigid connection portion comprises a glued coupling or a partly mechanical, partly glued coupling. The rigid connection portion may for example comprise a bonding material, such as an UV cross-linked polymer, ensuring the desired rigidity.

In an embodiment, the rigid connection portion comprises an integration portion integrating the balloon catheter and the inflation arrangement with each other, preferably to provide that the balloon catheter and the inflation arrangement form one unified structure.

The integration portion may for example comprise at least an integration between the syringe port and the lumen port, preferably to ensure the fluid connection between the barrel volume and the shaft lumen.

In a variation thereof the integration portion may be in the form of an integration between a portion of the balloon catheter and at least a portion of the inflation arrangement, wherein the portion on the balloon catheter does not include the lumen port and wherein the portion of the inflation arrangement does not include the syringe port.

The inflatable balloon may conveniently be is coaxially disposed about the distal shaft portion. Preferably, the distal shaft portion extends distally beyond the distal balloon end where the inflatable balloon is connected to the distal shaft portion at or adjacent to the distal balloon end.

Preferably, the balloon dilation system is adapted for being in a first balloon dilation system state, wherein the balloon is non-expanded and in a second balloon dilation system state wherein the balloon is expanded.

In the first balloon dilation system state, the plunger is advantageously in a retracted position, and the thruster is advantageously in a first thruster position. In the second balloon dilation system state, the balloon is advantageously expanded, and the plunger is advantageously in a deployed position, and the thruster is advantageously in a second thruster position.

In an embodiment, the second balloon dilation system state comprises a fully activated balloon dilation system state and a transition balloon dilation system state. In the fully activated balloon dilation system state, the balloon is fully expanded, the plunger is in a fully deployed position, and the thruster is in a fully activated position In the transition balloon dilation system state, the balloon dilation system is in a state between the first balloon dilation system state and the fully activated balloon dilation system state.

The barrel volume may comprise a liquid such as water, e.g. sterilized water and/or the inflation arrangement may comprise a closable liquid filling port, suitable for filling liquid into the barrel volume, optionally the liquid filling port form part of or is constituted by the syringe port.

In an embodiment, the barrel volume may comprise a liquid that has been prefilled with water at the factory e.g. via a filling opening, which thereafter may have been closed, and the entire balloon dilation system may have been sterilized.

In a variation thereof, the barrel volume may comprise a liquid that has been prefilled with water at the factory via a filling port, which thereafter has been closed with a plug, or the filling port may be a closeable liquid filling port as described below. Thereafter, the entire balloon dilation system may have been sterilized.

In a further variation thereof, the barrel volume is not prefilled but has a filling port for filling liquid into the barrel by the user, e.g. the physician.

It has been found that it may be difficult to ensure a sufficient and/or accurate fluid volume stored in the barrel in a balloon dilation system for storage, such as for storage in a packed and optionally sterilized condition for a longer time. A packed and optionally sterilized balloon dilation system wherein the barrel has been prefilled with fluid may therefore have a relatively short shelf time. Therefore, it may in any case be desired that the balloon dilation system comprises a closable liquid filling port, suitable for filling or after-filling liquid into the barrel volume.

The closeable liquid filling port may conveniently comprise a valve which automatically opens when a filler port of a filler unit, such as a filler syringe is inserted into the liquid filling port and wherein the valve automatically closes again when removing the filler port of the filler unit is withdrawn from the liquid filling port. The valve may e.g. be a one-way valve preventing back flow.

Advantageously, the rigid connection portion, such as the mechanical coupling, the mechanical-glued coupling or the integration portion has a rigidity that is sufficiently high to prevent at least the proximal shaft portion of the catheter shaft, such as the entire catheter shaft from rotating and translating relative to the inflation arrangement. Preferably, the rigid connection portion is capable of withstanding an axial displacement force of at least 1N, such as at least 10 N and/or a torsional stiffness (resistance to rotation about its own axis) of at least 10 N/mm, preferably at least 20 N/mm.

In an embodiment, the rigid connection portion has a rigidity that is sufficiently high to prevent at least the proximal shaft portion of the catheter shaft, such as the entire catheter shaft from rotating and translating relative to the inflation arrangement during ordinary handling of the balloon dilation system when performing a dilation procedure involving a inserting force not exceeding 10N.

The rigid connection portion, such as the mechanical coupling, the mechanical-glued coupling or the integration portion may preferably have a rigidity that is sufficiently high to provide that the rigid connection portion will be permanently damaged, such as fully or partially break before it will bend.

In an embodiment, the rigid connection portion is sufficiently strong to withstand a bending moment from the catheter shaft of at least 50 N/mm, such as a bending moment of at least 60 N/mm, such as a bending moment of at least 75 N/mm or higher.

Whereas it is common in the art to use Luer locks for fluidically coupling a syringe to a lumen of a balloon catheter, normally via a bendable tube, such standard Luer locks are generally adapted for ensuring tightness between the syringe and the lumen and has heretofore not been considered to be modified to be applied as part of a rigid coupling. Such prior art Luer lock connection does not provide a rigid connection and in particular not a connection that will be capable of handling or resisting the necessary torque.

The proximal shaft portion may in an embodiment extend from the rigid connection portion and to a proximal shaft portion distal end, wherein the proximal shaft portion distal end is located proximally to the distal balloon end. Preferably, the proximal shaft portion distal end is located proximally to a mid-length location of the inflatable balloon, wherein the mid-length location of the inflatable balloon is a middle location between the proximal balloon end and the distal balloon end.

Thereby the proximal shaft portion may extend to include a portion of the inflatable balloon.

In an embodiment, the proximal shaft portion is in the form of a rigid portion of the catheter shaft, optionally stiffened by a hypotube as described in detail below, the distal end of the hypotube may define the proximal end of the balloon. In some cases, the proximal shaft portion may comprise the proximal balloon end, and optionally the proximal balloon end may be fixed to the hypotube.

In an embodiment, the proximal shaft portion may extend from the rigid connection portion and to a proximal shaft portion distal end, wherein the proximal shaft portion distal end is located proximally to the balloon, and preferably with a distance to the balloon of up to 50% of the shaft length, such as up to 20% of the shaft length.

The proximal shaft portion may for example extend from the rigid connection portion and to a proximal shaft portion distal end located at least 70 mm from the rigid connection portion.

In an embodiment, the proximal shaft portion may extend from the rigid connection portion and to a proximal shaft portion distal end located at least 70 mm or even at least 90 mm from the rigid connection portion.

The balloon catheter shaft length may advantageously be between 80-280 mm, such as 90-250 mm, such as 100-200 mm, such as 110-170 mm.

In an embodiment, the proximal shaft portion is defined as the length of the balloon catheter shaft extending from the rigid connection portion to the proximal end of the balloon.

The proximal shaft portion advantageously has a length of from 50 mm to 250 mm, such as form 70 mm to 200 mm, such as from 80 mm to 150 mm, such as from 90 mm to 120 mm.

In an embodiment, the proximal shaft portion comprises a steel tube with an outer diameter of from 1-4 mm such as from 1,5-3 mm, such as from 1,7-2,5 mm.

The distal shaft portion may in an embodiment be defined as the length of the balloon catheter shaft extending from the proximal shaft portion distal end to the balloon distal end. The distal shaft portion may then conveniently have a length of from 10-50 mm, such as from 20-40 mm.

In an embodiment, the distal shaft portion comprises a steel rod or a steel tube, preferably having an outer diameter of from 0,5-1,5mm.

In an embodiment, the distal shaft portion comprises a malleable steel rod that may optionally have an internal lumen.

In an embodiment, the distal shaft portion comprises a flexible wire, such as a metal wire, polymer wire or a carbon or glass fiber wire, preferably with a diameter of less than 0,7 mm.

In an embodiment, the distal shaft portion may comprise several elements and may comprise a proximal malleable end and a flexible distal end to allow forming of a balloon angle relative to the shaft and yet having a flexible balloon tip.

In an embodiment, the distal shaft portion comprises a rigid steel rod with a pre-shaped curve or bend angle adopted for insertion into a specific passageway.

In an embodiment, the distal shaft portion extents distally from the balloon distal end and preferably comprises an atraumatic tip, such as an olive tip or a bulbous tip.

In an embodiment, the balloon has a cylindrical portion with a diameter of from 3-10 mm, such as from 4-8 mm, such as from 4-7 mm.

In an embodiment, the balloon has a cylindrical portion with an axial length of from 10-40 mm, such as from 12-30 mm, such as from 15-25 mm.

In an embodiment, the displaceable liquid volume in the syringe barrel is between 0,5 and 5 ml, such as from 1-3 ml, such as from 1-2 ml.

In an embodiment, the internal diameter of the syringe barrel is between 3 and 8 mm, such as from 4-6 mm. The plunger force required to deliver 12 bar in the balloon may preferably be lower than 50 N and optimally lower than 30 N.

As mentioned above, the thruster and the grasping portion may be arranged with an optimized architecture ensuring a relatively simple and uncomplicated handling by a physician.

In an embodiment, the thruster is displaceable, preferably axially displaceable relative to the grasping portion for filling the catheter balloon with fluid from the barrel and optionally for withdrawing the fluid from the catheter balloon when the dilation is to be terminated.

Advantageously, the inflation arrangement comprises at least one linear thruster track for displaying the thruster. The thruster may comprise at least one track grip adapted for engage with and slide along the at least one thruster track. Optionally, the at least one linear thruster track is parallel with the syringe axis, which may ensure a filling of fluid into the catheter balloon without undue force is required.

In an embodiment, the thruster is partially and displaceable located in a thruster housing, wherein the thruster housing optionally is integrated with or coupled to the barrel, such as integrated with or coupled to a proximal end portion of the barrel.

The thruster may have a distal thruster end connected to or integrated with a proximal end of the barrel, such as a proximal end wall of the barrel, wherein the thruster end/barrel proximal end comprises a thruster/barrel passage, wherein the plunger rod is displaceable passing through the thruster/barrel passage. The plunger rod may conveniently have a protuberance located in the thruster to prevent the plunger from slipping through the thruster/barrel passage and into the barrel.

Thereby, when mowing the thruster in proximal direction, the plunger cannot pass through the thruster/barrel passage, and thereby the plunger will be pulled in proximal direction with the thruster via pulling in the protuberance of the plunger.

In an embodiment, the grasping portion protrudes outwards from an outer annular wall face of the barrel and/or outwards from an outer wall face the thruster housing, optionally the grasping portion protrudes radially (e.g. about 90° to the syringe axis) or with an angle to the syringe axis of at least 45° from the outer annular wall face of the barrel and/or outwards from the outer wall face the thruster housing.

Advantageously, at least a head portion of the thruster is located outside the thruster housing to ensure a simple activation of the thruster. Preferably at least a head portion of the thruster is located outside the housing even when the thruster is in the second thruster position.

The thruster housing may conveniently comprise a thruster guide section, comprising the at least one linear thruster track.

In an embodiment, the thruster housing comprises a thruster lock adapted for temporally locking the thruster in the second thruster position.

In an embodiment, the thruster is engaged with the plunger or the barrel via a gearing arrangement, wherein the gearing arrangement is arranged to reduce the force required to deploy the thruster from the first thruster position to the second thruster position, preferably the gearing arrangement is engaged with the thruster and the plunger head to provide that the thruster is pivotally movable relative to the plunger.

Thereby, the force required to deflate the balloon by pressing fluid via the shaft lumen into catheter balloon may be reduced, which is in particular desired where the physician has to perform several dilations procedures over a short time slot.

In an embodiment, the inflation arrangement comprises an inflation arrangement housing and optionally, the grasping portion is located at or form part of the inflation arrangement housing,

In an embodiment, the inflation arrangement is coupled to or integrated with the balloon catheter in the rigid connection portion via the inflation arrangement housing.

In an embodiment, the inflation arrangement is coupled to or integrated with the balloon catheter in the rigid connection portion via a catheter connector forming part of the rigid connection portion.

The catheter connector may in an embodiment be a permanent part of the balloon catheter, e.g. located at the most proximal end of the balloon catheter and comprising the lumen port fluidically connected to the shaft lumen.

In an embodiment, the catheter connector further comprises means, such as a snap-lock, a thread or similar connection means for mechanical coupling of the catheter connector to the inflation arrangement.

As described further below, the catheter connector may in an embodiment comprise an access port connected to a working channel of the balloon catheter.

In an embodiment, the thruster is pivotally engaged with or connected to the plunger or the barrel, wherein a pivoting motion of the thruster comprises pivoting the thruster relative to the inflation arrangement housing, whereby the thruster effectuates a motion of one of the plunger and the barrel.

In an embodiment, the thruster is partially and displaceable located in a thruster housing, wherein the thruster housing optionally is integrated with or coupled to the barrel, such as integrated with or coupled to a proximal end of the barrel.

In an embodiment, the fluid connection between the barrel volume and the shaft lumen comprises an intermediate flexible tube section located inside or outside the inflation arrangement housing.

In an embodiment, the thruster and the grasping portion are movable relative to each other via a threaded engagement to move the plunger and the barrel relative to each other.

In an embodiment, the thruster comprises a thumb ring. The thruster ring is preferably adapted to be pushed and optionally pulled by the thumb of an operator.

In an embodiment, the thruster form part of or is constituted by the plunger.

In an embodiment, the grasping portion is connected to or integrated with the barrel, and wherein the thruster is operably coupled to the plunger rod and/or the plunger head.

In an embodiment, the syringe port forms part of the barrel and wherein the syringe port is directly connected to the lumen port, preferably without any other fluid connection parts in between.

In an embodiment, the rigid connection portion directly interconnects the barrel and the catheter shaft, preferably the rigid connection portion comprises a mechanical coupling and wherein the mechanical coupling is establishing a rigid coupling directly between the barrel and the catheter shaft.

In an embodiment, the rigid connection portion directly interconnects the barrel and the catheter shaft.

The syringe axis and the shaft axis may conveniently be parallel.

In a variation thereof, the syringe axis and the shaft axis have an angle to each other of from 0 to 30°, such as from 0 to 20°.

The syringe may preferably be in the form of an inverted syringe, wherein the grasping portion is engaged with, connected to or forms part of the plunger rod and wherein the thruster is operably coupled to the barrel for pushing the barrel over the plunger rod in a distal direction towards the balloon catheter to inflate the balloon.

It has been found that the inflation arrangement may be very compact where the syringe may preferably be in the form of an inverted syringe.

In an embodiment, the plunger rod comprises a plunger lumen fluidically connecting barrel volume with syringe port.

The rigid connection portion may in an embodiment directly interconnect the plunger rod and the catheter shaft. Preferably, the rigid connection portion comprises a mechanical coupling and wherein the mechanical coupling is establishing or forming part of a rigid coupling directly between the plunger rod and the catheter shaft.

In an embodiment, the catheter shaft has a distal end and comprises a shaft rod that extends from the rigid connection portion and along the entire shaft length to the distal end of the catheter shaft, wherein the shaft rod preferably forms an internal part of the catheter shaft.

In an embodiment, the shaft lumen in at least a length portion of the catheter shaft is in the form of a gap between an outer surface of the shaft rod and an inner surface of a fluid connection tube disposed concentrically about the rod.

Advantageously, the shaft lumen in at least a length portion of the catheter shaft is in the form of a channel inside the shaft rod.

In an embodiment, the shaft rod has a distal end with a curved portion located at the distal shaft portion and proximally to the distal balloon end, wherein the distal shaft portion preferably extends from the distal end of the catheter shaft and at least to a mid-length location of the inflatable balloon, wherein the mid-length location of the inflatable balloon is a middle location between the proximal balloon end and the distal balloon end.

In an embodiment, the distal shaft portion extends from the distal end of the catheter shaft and at least to the proximal balloon end, such as to 1 cm distal to the proximal balloon end, such as to a distal end of a hypotube.

In an embodiment, where the catheter shaft comprises a hypotube the hypotube defines the proximal shaft portion and the distal shaft portion is in the form of the portion of the catheter shaft located distally to the hypotube.

Preferably, the entire shaft rod has a stiffness that prevents elastic and/or plastic deformation of more than 2 mm in any direction and at any point of the catheter shaft during a normal procedure involving an axial force on the shaft rod not exceeding 10N, and a transverse force at any point of the shaft rod not exceeding 1N

In an embodiment, the shaft rod has a distal end and a stiffness sufficiently high to prevent the distal end of the shaft rod from deflecting beyond 5 mm, preferably 1 mm or less when applying a force F at the distal end of the shaft rod, wherein F is up to 1N, e.g. using the single sided clamp method.

Advantageously, the shaft rod has a distal end, and wherein the distal end of the shaft rod is malleable and preferably adapted for being shaped by a physician. Thereby, the physician may in a relatively simple way ensure that the distal end of the shaft rod is adapted to the person under treatment.

The desired degree of malleability and/or flexibility of the distal end of the shaft rod depends in practice on the anatomic passageway to be dilated.

Where the anatomic passageway to be dilated is a Sinus passageway it is normally desired that the shaft rod is relatively stiff. Where a navigation sensor, such as a clamp sensor as described below, is used, the navigation sensor will normally be placed proximally e.g. at the proximal shaft portion. To ensure navigation with a high accuracy, it is desired that the shaft rod is very stiff, so that the position of the tip of the shaft rod is determinable according to where the sensor is located with a high accuracy. Typically, the tip I in this situation not bendable by a user.

Where the anatomic passageway to be dilated is a Sinus passageway and the navigation sensor is located in the distal tip close to the catheter balloon, such as adjacent to the catheter balloon, the distal shaft rod must only be stiff enough for the procedure to be performed so that it does not plastically deform during the procedure, but it may elastically bend, e.g. with a deflection <5mm. Here, the distal rod can be moldable by the user, possibly by using a bending tool.

Where the anatomic passageway to be dilated is a Eustachian tube, the distal end may advantageously be as little stiff as possible, and preferably there must be high flexibility to prevent undesired forces from being transferred from the proximal shaft to the Eustachian tube. The distal rod end may, for example, be in the form of a spring wire e.g. of stainless steel or nitinol that may be pre-bent to e.g. 45 degrees and preferably is less than 1mm in diameter.

In an embodiment, at least a part of the proximal shaft portion comprises a shaft hypotube and wherein the shaft hypotube preferably is located to form an annular reinforcement of at least the part of the proximal shaft portion.

Advantageously, the shaft lumen at least in the distal shaft portion is located between an outer surface of the shaft rod and an inner surface of the shaft hypotube and wherein the proximal end of the balloon is bonded to the distal end of the shaft hypotube and wherein the distal end of the balloon is bonded to a distal end of the shaft rod.

In an embodiment, the balloon catheter comprises a working lumen suitably arranged for insertion of at least one of a guide wire, a light fiber, an electromagnetic sensor or for delivering a liquid substance, or to facilitate suction, and wherein the balloon catheter comprises an access port to the working channel.

Advantageously, at least a distal length section of the working lumen is located inside and extends lengthwise in the shaft rod.

The access port to the working lumen may conveniently be located at, and form a proximal end, of the working lumen. Preferably at least a distal length section of the working lumen is coaxial with the shaft axis.

This architecture of the working channel /shaft may be referred to as an "over-the-wire architecture".

In an embodiment, the access port to the working channel is located distally to the lumen port at an acute angle relative to the catheter center axis.

This architecture of the working channel/shaft may be referred to as a "rapidexchange architecture".

In an embodiment, the balloon catheter comprises a catheter connector arranged for connecting the inflation arrangement and the balloon catheter and forming part of the rigid connection portion. Optionally, the catheter connector comprises a connector working lumen forming part of the working lumen with a proximal access port into the connector working lumen. The catheter shaft may comprise a shaft working lumen forming part of the working lumen and located to form an extension of the connector working lumen.

The catheter connector may preferably have a T-shaped architecture or a bifurcated architecture. In an embodiment, the catheter connector forms part of or is connected to a housing containing at least a part of the inflation arrangement.

In an embodiment, the catheter connector has a bifurcated architecture, with a distal catheter connector branch, a catheter shaft branch and a working channel branch, wherein the catheter shaft branch and the working channel branch are located proximally to the distal catheter connector branch and wherein the access port is located in a proximal end of the working channel branch.

In an embodiment, the catheter connector has a T-shaped architecture wherein the fluid connection between the barrel volume and the shaft lumen comprises a fluid passage orthogonal to the shaft axis.

In an embodiment, at least a part of the catheter connector forms part of or constitutes the rigid connection portion, preferably at least the catheter shaft branch and the distal catheter connector branch form part of or constitute the rigid connection portion.

The lumen port may be located in the catheter connector and preferably comprising a fluid passage orthogonal to the shaft axis to the shaft axis.

In an embodiment, the lumen port is located at a proximal end of the catheter shaft and has a lumen port length coaxial with the shaft axis.

In an embodiment, the syringe port extends transverse to the syringe axis across a wall of the syringe, e.g. across the wall of the barrel.

In an embodiment, the syringe port extends parallel to the syringe axis and forms a distal tip of the syringe and wherein the syringe is connected directly to the catheter shaft.

In an embodiment, the proximal shaft portion comprises a visually marked area adapted for clamping on a clamp-type sensor or wherein the proximal shaft portion comprises a clamp-type sensor clamped on to the proximal shaft portion. The clamp-type sensor may advantageously be electrically connected to or is electrically connectable to an electromagnetic or visual navigation system.

By applying such clamp-type sensor, the handling of the balloon dilation system, including guiding the catheter balloon into a correct location of the anatomic passageway may be even simpler for the physician, since the clamp-type sensor may further increase the physician's perception of the location of the catheter balloon.

In an embodiment, at least a length section of the proximal shaft portion comprises an externally located steel tube, preferably having a diameter of at least 3 mm, a length of at least 10 mm and a wall thickness of at least 0.2 mm.

The steel tube may comprise or constitute a visually marked area adapted for clamping on a clamp-type sensor fur increasing the perception of the physician, In a variation thereof the proximal shaft portion may comprise a clamp-type sensor clamped on to the proximal shaft portion, wherein the clamp-type sensor conveniently is electrically connected to or is electrically connectable to an electromagnetic or visual navigation system.

In an embodiment, the inflation arrangement comprises a protruding cylindrical pin, wherein the protruding cylindrical pin is adapted for clamping on a clamp-type sensor or wherein the protruding cylindrical pin comprises a clamp-type sensor clamped on to the protruding cylindrical pin, wherein the clamp-type sensor is electrically connected to or is electrically connectable to an electromagnetic or visual navigation system. Preferably, the cylindrical pin has a diameter of at least 3 mm and a length of at least 5 mm.

Where the balloon dilation system comprises a clamp-type sensor, the inflation arrangement may conveniently comprise a permanently mounted electromagnetic sensor or emitter.

In an embodiment, the balloon dilation system comprises a filling port for filling liquid into the syringe and into the balloon catheter while the syringe and the balloon catheter are fluidically connected.

In an embodiment, the rigid connection portion comprises a glued connection between the proximal shaft portion and the syringe, preferably the lumen port is glued directly to the syringe port.

In an embodiment, the syringe comprises a spring element, wherein the spring element is located between the thruster and the plunger.

The thruster may in an embodiment be operably coupled to drive the plunger in a distal direction for inflation of the balloon.

In an embodiment, the thruster is configured for being moved in a distal direction for inflation of the balloon.

In an embodiment, the plunger is configured for being moved in a distal direction towards the balloon and parallel to the balloon catheter center axis for inflation of the balloon.

In an embodiment, the thruster is shaped to be pushed by the thumb of an operator.

Advantageously, the balloon dilation system is configure to be operated with one hand, with one thumb of the one hand on the thruster and at least two fingers of the same one hand on the grasping portion and wherein the grip position of the one hand is the same during insertion of the balloon and during inflation of the balloon.

The invention also comprises a balloon dilation kit of parts.

The balloon dilation kit of parts comprises
a balloon catheter comprising;
   a catheter shaft having a shaft length, a shaft axis, a proximal shaft portion, a distal shaft portion and a shaft lumen,
   an inflatable balloon located at the distal shaft portion, the balloon having a proximal balloon end and a distal balloon end and the balloon being in fluid connection with the shaft lumen, and
   a lumen port at the proximal shaft portion in fluid connection with the shaft lumen,
an inflation arrangement comprising;
   a syringe comprising a barrel having a barrel volume, a syringe axis, and a plunger with a plunger rod and a plunger head,
   a syringe port in fluid connection with the barrel volume,
   a grasping portion engaged with, connected to or forming part of one of the plunger and the barrel,
   a thruster engaged with, connected to or forming part of another one of the plunger and the barrel,
wherein the barrel volume is in fluid connection with the shaft lumen,
wherein the grasping portion is located with a first distance d1 to the proximal balloon end and the thruster is located with a second distance d2 to the proximal balloon end and wherein the second distance d2 is larger than the first distance d1; and/or
wherein the inflation arrangement has a distal end defined by the syringe port and wherein the grasping portion is located distally to the thruster, and
wherein the balloon catheter and the inflation arrangement are adapted for being directly coupled to or integrated with each other to form a rigid connection portion.

The balloon dilation kit of parts may comprise a balloon dilation system according to any one of the preceding claims, wherein at least one element of the balloon dilation system is separate or separated from at least one other element of the balloon dilation system.

In an embodiment, the kit comprises at least two balloon catheters having distal shaft portions that differ from each other, such as distal shaft portions that differs from each other, preferably a first of the at least two balloon catheter has a fixed length section pre-bent to have a first curved shape and or angle relative to the proximal shaft portion and a second of the at least two balloon catheter has a fixed length section pre-bent to have a second curved shape and or angle relative to the proximal shaft portion and a second of, wherein the first curved shape and or angle relative to the proximal shaft portion differs from the second curved shape and or angle relative to the proximal shaft portion.

In an embodiment, the proximal shaft portion of the balloon catheter comprises a visually marked area adapted for clamping on a clamp-type sensor and wherein the kit comprises a clamp sensor adapted for being clamped to the visually marked area of the proximal shaft portion, preferably at least a length section of the proximal shaft portion comprises an externally located steel tube comprising the visually marked area.

In an embodiment, the inflation arrangement comprises a protruding cylindrical pin and wherein the kit comprises a clamp sensor adapted for being clamped to the protruding cylindrical pin comprises a clamp-type sensor clamped on to the protruding cylindrical pin.

Advantageously, the balloon dilation kit comprises an electromagnetic sensor configured for being inserted into a working channel of the balloon catheter.

Advantageously, the balloon dilation kit comprises a light fiber configured for being inserted into a working channel of the balloon catheter.

The parts of the balloon dilation kit may preferably be packed in one or more packagings and sterilized, preferably all parts of the balloon dilation kit are packed in one packaging and sterilized together.

The invention also comprises a first method for balloon dilation of an anatomic passageway in the head of a person.

The first method for balloon dilation of an anatomic passageway in the head of a person, the method may advantageously comprise using the balloon dilation system as described above.

The first method for balloon dilation of an anatomic passageway in the head of a person comprises
a) Optionally shaping the distal end of the balloon catheter
b) with one hand grasping the grasping portion, moving the catheter shaft forward into the nostril of a person
c) with the same one hand grasping the grasping portion, moving the distal shaft portion transverse to the insertion direction to push the balloon into a Sinus passageway or into the Eustachian Tube
d) with the thumb of the same one hand, pushing the thruster in a distal direction towards the balloon and relative to the grasping portion for inflating the balloon to thereby dilate the passageway,
e) optionally deflating the balloon by retracting or releasing the thruster,
f) removing the balloon catheter from the passageway and from the natural opening.

In a variation thereof, the plunger is moved in a distal direction towards the balloon and parallel to the balloon catheter center axis for inflation of the balloon.

The invention also comprises a second method for balloon dilation of an anatomic passageway in the head of a person.

The second method for balloon dilation of an anatomic passageway in the head of a person, the method may advantageously comprise using the balloon dilation system as described above.

The second method for balloon dilation of an anatomic passageway in the head of a person comprises
a) Clamping a clamp-type navigation sensor onto a part of the Balloon Dilation System
b) Calibrating the navigation sensor to track the most distal tip of the balloon catheter
c) with one hand grasping the grasping portion, moving the catheter shaft forward into the nostril of a person
d) with the same one hand grasping the grasping portion, moving the distal shaft portion transverse to the insertion direction to push the balloon into a Sinus passageway or into the Eustachian Tube
e) confirm placement of the balloon in the passageway by means of the navigation sensor and a navigation system.
f) with the thumb of the same one hand, pushing the thruster in a distal direction towards the balloon and relative to the grasping portion for inflating the balloon to thereby dilate the passageway,
g) optionally deflating the balloon by retracting or releasing the thruster,
h) removing the balloon catheter from the passageway and from the natural opening.The invention further comprises following balloon dilation system with a balloon catheter and an inflation arrangement with,

a balloon catheter comprising
   a catheter shaft having a shaft length, a shaft axis, a proximal shaft portion, a distal shaft portion and a shaft lumen,
   an inflatable balloon located at the distal shaft portion, the inflatable balloon having a proximal balloon end and a distal balloon end and the inflatable balloon being in fluid connection with the shaft lumen, and
   a lumen port at the proximal shaft portion in fluid connection with the shaft lumen, and
an inflation arrangement comprising
   a syringe comprising a barrel having a barrel volume,
   a syringe port in fluid connection with the barrel volume,
   a grasping portion engaged with, connected to or forming part of the barrel,
   a plunger with a plunger rod and a plunger head,
   a thruster slidably coupled to the plunger rod,
   a spring positioned between part of the plunger and part of the thruster,
   wherein an axial force applied to the thruster in a distal direction is transferred through the spring to the plunger and wherein the thruster can be moved distally relative to the plunger by compression of the spring.

For an inflation arrangement it may be advantageous to have a spring element that reduces the risk of overinflating the balloon with too high pressure. A simple way to control the pressure from an inflation arrangement may be to place a spring between the plunger and an added thruster onto which an operator will apply the force.

In an embodiment, the spring is locked and preloaded in a first compressed length between part of the plunger and part of the thruster. It may be advantageous that the thruster and the plunger moves in unison in a first state when pumping liquid out of the barrel volume and into the balloon catheter and wherein the thruster only moves distally relative to the plunger when a certain force has been applied, corresponding to a certain hydrostatic pressure in the barrel volume.

It would be advantageous to have a long spring with a very low spring rate that is precompressed substantially to reach a target force at the preloaded first compressed length. A long spring with a low spring rate reduces influences from spring tolerances. Also, changes in compression length during pumping of liquid will have little influence on the spring force that acts onto the plunger and influences the hydrostatic pressure that is delivered to the balloon.

In an embodiment, the first compressed length is at least 20% shorter than the free length of the spring, such as 30% shorter or even 40% shorter.

In an embodiment, the free length of the spring is longer than 70mm, such as longer than 90 or longer than 110mm.

In an embodiment, the spring rate is lower than 3N/mm, such as lower than 1N/mm or such as lower than 0,7mm.

In an embodiment, the plunger has a locking feature that prevents axial movement of the thruster in a proximal direction. An axial lock between the two parts is needed to assemble and hold the spring in a preloaded state between the plunger and the thruster.

In an embodiment, a hole in the thruster is configured to slide axially over the plunger rod from a proximal position to a distal position by compression of the spring.

In an alternative embodiment, the plunger rod is hollow and part of the thruster is configured to slide inside a hollow plunger rod from a proximal position to a distal position by compression of the spring.

Most inflation arrangements have means for indicating the pressure. The level of compression of the spring in this invention may be used as an indication of the applied force to the plunger and thereby the hydrostatic pressure in the barrel volume. A distance between visual markings on the plunger and on the thruster may be used to indicate if the pressure is within an acceptable range.

In an embodiment a first pressure dial marking is on the plunger. The first pressure dial marking may one or more visible markings such as a ring element or a colored ring marking or a groove. The first pressure dial marking may be an o-ring seal on the plunger head. The first pressure dial marking may located on the most proximal end of the plunger rod. The first pressure dial marking may be a locking ring on the plunger rod.

In an embodiment, the second pressure dial marking is on the thruster. The second pressure dial marking may be one line or dot or may be several visible markings that are distanced axially. If the first pressure dial marking is to be visible, it may be advantageous to have one or more openings on the side wall of the truster or the thruster may be partially or fully transparent.

The first pressure dial marking on the plunger will have a first distance to the second pressure dial marking when the thruster is in a first state with no force applied to it and the first pressure dial marking will have a second distance to the second pressure dial marking, when the thruster is in a second state where such axial force is applied to it, that the spring is further compressed,

For ease of use, it may be advantageous to allow the operator to fully deploy a thruster to an end-stop rather than observing a pressure indicator carefully during pumping and inflation. In an embodiment of the invention, the system is designed to eliminate overinflation with too high pressure. This can be achieved by having an inflation arrangement that is configured to pump only a limited liquid volume and wherein the pumpable limited liquid volume is only slightly more liquid than required to fully inflate and pressurize the balloon catheter. When the thruster is pressed to an end-stop, the balloon is fully inflated and pressurized, the surplus liquid volume will prevent the plunger from reaching its most distal position and the spring will be compressed. A variation of the surplus liquid volume caused by tolerances, may influence the compression length of the spring and thereby the hydrostatic pressure in the barrel. With a very low spring rate, the changes in spring compression and changes in hydrostatic pressure may be negligible. For such an inflation arrangement, it may be enough to ensure that the spring is just compressed slightly, as this may indicate that the pressure is ok.

In an embodiment of the invention, the displaceable liquid volume from the barrel by movement of the plunger is less than 10 times the internal volume of the balloon catheter, such as less than 5 times or such as less than 2 times the internal volume of the balloon catheter.

In an embodiment of the invention, the thruster has an end-stop surface that has contact with a counter stop surface placed on the barrel or a structure mounted on the barrel, when the thruster is an a distal end-stop position.

In an embodiment, the inflation arrangement may comprise a releasable lock for locking the thruster relative to the barrel in exactly one position being at the most distal position of the thruster.

### BRIEF DESCRIPTION OF PREFERRED EMBODIMENTS AND ELEMENTS OF THE INVENTION

The above and/or additional objects, features and advantages of the present invention will be further elucidated by the following illustrative and non-limiting description of embodiments of the present invention, with reference to the appended drawings.

The figures are schematic and are not drawn to scale and may be simplified for clarity. Throughout, the same reference numerals are used for identical or corresponding parts.

Figs. 1a-1g show a first embodiment of a balloon dilation system 100 according to the invention in different stages.

Figs. 2a-c show a second embodiment of a balloon dilation system 200 according to the invention.

Figs. 3ae show a third embodiment of a balloon dilation system 300 according to the invention in a disassembled and an assembled state.

Figs 4a-4b show a fourth embodiment of at balloon dilation system 400 according to the invention in different stages.

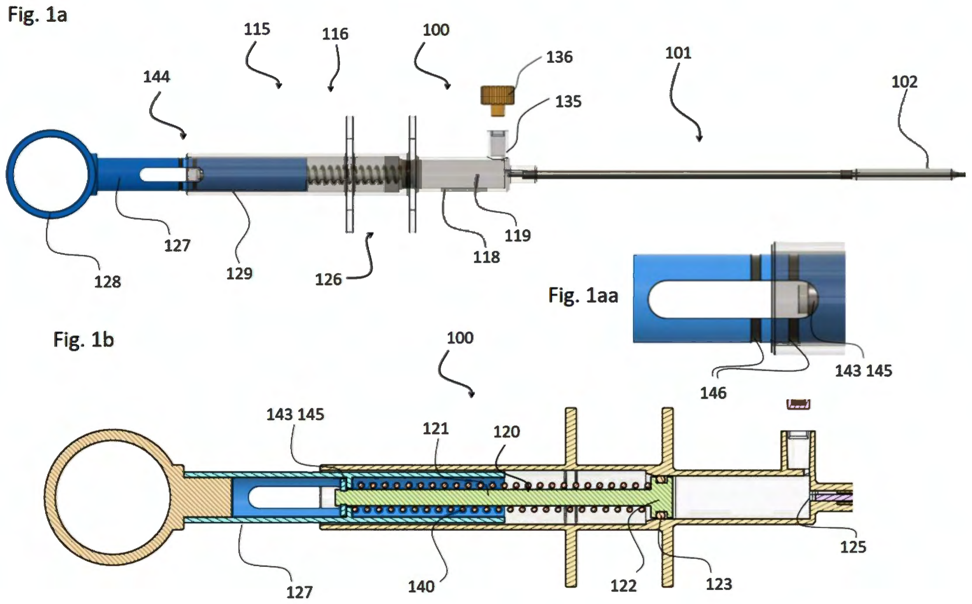

Figs. 1a and 1b show a side view and section view of the first embodiment of the balloon dilation system 100 in a first stage where the system is ready to be filled with liquid as part of the preparation. The balloon catheter 101 has a balloon 102 shown in a non-expanded state.

The inflation arrangement 115 comprises a syringe 116 with a barrel 118 with a barrel volume 119 and a syringe port 125. The barrel 118 has a grasping portion 126 and a filling port 135 with a filling port plug 136. The syringe 116 further comprises a plunger 120 with a plunger rod 121 and a plunger head 122 with a plunger head seal 123. A thruster 127 with a head portion of thruster 128 in a thruster housing 129.

The shaft rod 109 is shown in a straight configuration before being bend or shaped by the operator. Liquid may be poured in, pushed in or pulled into the barrel 118 through the filling port 135. The balloon 102 may be collapsed and folded and have a protective sleeve on during filling of the barrel 118, to keep liquid from entering the balloon during the filling process. A filling port plug 136 may be used to close the filling port 135 after filling of liquid. The thruster 127 is in a first position. The plunger 120 is in a fully retracted position. The thruster 127 has a ring for e.g. the thumb of the operator. The thruster 127 is guided inside a thruster housing 129 which in this example is a cylindrical proximal extension of the barrel 118. The grasping portion 126 is comprised of the outer surface of the barrel 118 and two flanges protruding from the barrel 118 and where an operator may have e.g. the index finger between the two flanges over the barrel 118 and e.g. the middle finger between the two flanges under the barrel 118. A spring 140 on the plunger rod 121 is pre-compressed to a first spring length between the plunger head 122 and a wall in the thruster 127. Part of the proximal end of the plunger rod 121 passes through a hole in the wall of the thruster 127 and has a plunger lock ring 143 that prevents it from passing through the thruster wall hole in a distal direction. As seen best in section view fig. 1b and in detail view fig 1aa, the plunger lock ring 143 acts as a first pressure dial 145 of a pressure indicator 144 and is visible to the operator through the thruster 127 and through the thruster housing 129. For manufacturing reasons, it may be more favorable to have the plunger head 122 as a separate component to be mounted onto the plunger rod 121 using e.g. a threaded connection and in such a case, the plunger rod 121 may have an increased diameter in the proximal end with an e.g. colored ring instead of the lock ring. In this example the thruster 127 has a slit and the thruster housing 129 is transparent. A second pressure dial 146 comprises two lines at the window of the thruster 127 is visible to the operator. When the spring 140 has the first pre-compressed spring length, the first pressure dial 145 is distal to any part of the second pressure dial 146. The thruster 127 and plunger 120 will move together in unison as long as the internal hydrostatic pressure inside the barrel 118 is below a pressure setpoint (such as a selected threshold). The setpoint could be e.g. 10 or 12 atm. When the pressure is at or above the setpoint, the spring 140 will be compressed further and the plunger 120 will move to a new position relative to the thruster 127. The spring forces are known at different spring compression lengths, and specific positions of the plunger 120 relative to the thruster 127 may therefore be an indicator for the force that the plunger head 122 applies to the liquid in the barrel 118 and for a known internal cross-sectional area in the barrel 118, the relative position of the plunger 120 relative to the thruster 127 may be a good indicator for the internal hydrostatic pressure in the barrel 118. In other embodiments, the first pressure dial 145 on the plunger rod 121 may be different, such as a ring, one or more dots or lines on the plunger rod 121 or the end surface or an edge on the plunger rod 121. In other embodiments, there may be a different way of viewing changes in the relative position between the plunger 120 and the thruster 127. In some examples, the first pressure dial 145 on the plunger rod 121 may be visible through a transparent portion of the thruster 127 and in some examples, there may be a slit or a window in a non-transparent thruster housing 129. In other embodiments according to the invention, the second pressure dial may include several lines that are axially spaced and have written pressure values from 8 bar to 12 bar such than the operator can depress the truster to a desired balloon pressure. In another embodiments according to the invention, the second pressure dial may be a single marking at one axial position such as one single line, ring, grove or a dot and wherein the any position of the first pressure dial distal from the second pressure dial indicates too low pressure when the thruster is fully deployed, and wherein any position of the first pressure dial proximal from the second pressure dial indicates that the pressure is OK, when the thruster is fully deployed.

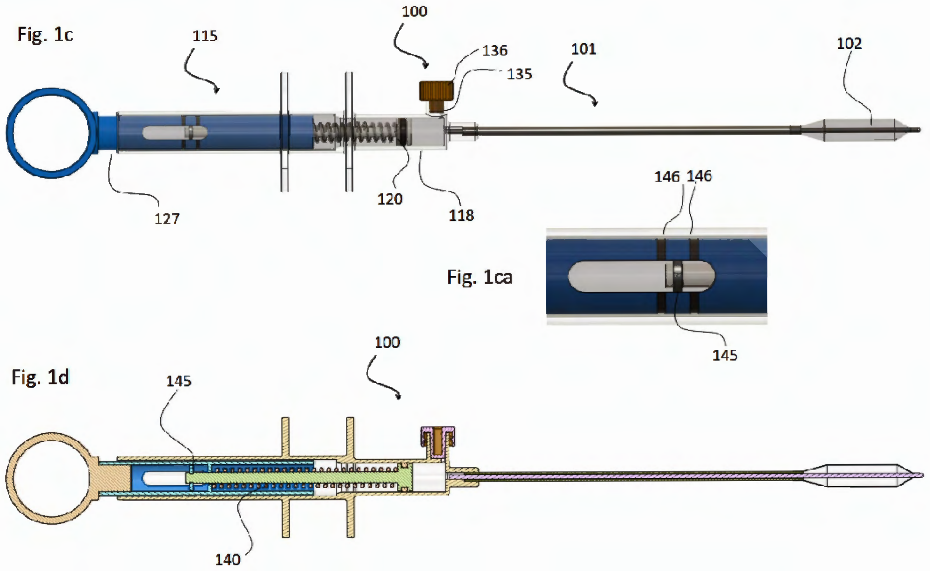

Figs. 1c and 1d show a side and section view of the first embodiment in a second stage. The balloon dilation system 100 is filled with liquid, wherein the filling port 135 is closed by the filling port plug 136 the balloon 102 is expanded and pressurized, the plunger 120 is deployed into the barrel 118 and the thruster 127 in a second position. The exact position of the plunger relative to the barrel 118 when the balloon 102 is completely expanded may vary depending on tolerances in the system, the liquid filling accuracy, amount of trapped air etc. For a system with high tolerances on the internal volumes and liquid volume, a position of the plunger relative to the barrel 118 provides no meaningful information and, in such systems, the operator will need to monitor the pressure indicator 144 during movement of the thruster 127, to ensure correct system pressure and to avoid under or over pressure in the system during the procedure. The applied force from the operator onto the thruster 127 will initially move the thruster 127 and the plunger 120 forward into the barrel 118 in unison until the non-compliant or semi-compliant balloon 102 has reached its expanded diameter. Further force applied to the thruster 127 causes the thruster 127 to be moved further forward relative to the plunger 120 and resulting the spring 140 in being compressed to a second shorter spring length. As can be seen in detail in fig. 1ca, the first pressure dial 145 is aligned between the two lines of the second pressure dial 146 to indicate to the operator, that the desired force and thereby the desired hydrostatic pressure of e.g. 12atm pressure in the barrel 118 and in the balloon 102 is reached.

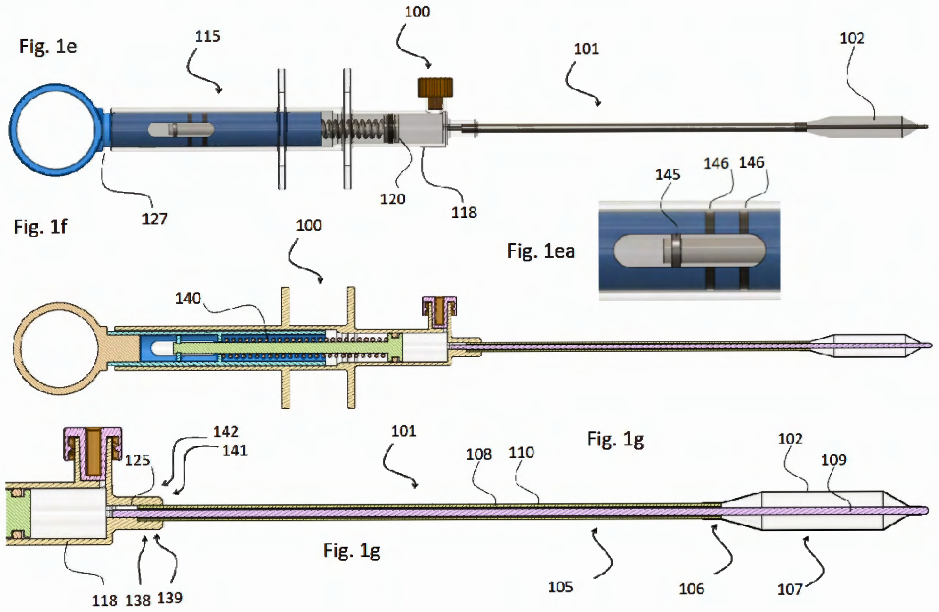

Figs. 1e, 1ea, and 1f show a side, detail and section view of the first embodiment in a third stage wherein the balloon 102 is expanded, the plunger 120 is deployed and the thruster 127 has been pushed too hard and too far distally by the operator. The spring 140 is compressed to a third even shorter length and the first pressure dial 145 is positioned proximal from the most proximal line of the second pressure dial 146 to indicate to the operator, that the force and the pressure is too high.

Fig. 1g shows a detailed section view of the balloon catheter 101 in an expanded state. The catheter shaft 103 with a proximal shaft portion 105 with a shaft portion distal end 106 and a distal shaft portion 107, a shaft rod 109 and a shaft hypotube 110. The catheter shaft 103 has a shaft lumen 108 that fluidically connects the balloon 102 to a lumen port 111. The catheter shaft 103 is connected directly to the distal end of the barrel 118. The shaft rod 109 and the shaft hypotube 110 are both inserted directly into the barrel 118 at the syringe port 125 and at least the shaft hypotube 110 has a glued connection to the barrel 118 to provide a mechanical coupling 138 to the barrel 118 and a fluid coupling 139 to the barrel volume 119 and defines an integration portion 141. A rigid connection portion 142 is provided by the insertion of a portion of the catheter shaft 103 directly into the distal end of the barrel 118 and wherein the insertion depth of the catheter shaft 103 into the barrel 118, the thickness of the material of the barrel 118 that surrounds the inserted portion of the catheter shaft 103, and the fit or bonding radially between the internal surface of the hole in the barrel 118 and the outer surface of the inserted proximal portion of the catheter shaft 118 determined the robustness and stiffness of the rigid connection portion 142. The insertion depth of the catheter shaft 103 into a hole may need to be more than 10mm and the thickness of a material surrounding the insertion hole may need to be at least 1mm. The shaft rod 109 may be fixed to the barrel 118. The shaft rod 109 may, in other embodiments not be fixed to the barrel 118 but needs to be prevented from rotating and translating along its own center axis relative to the shaft hypotube 110. The shaft rod 109 may be fixated to the shaft hypotube 110 at one or more locations e.g. by a friction fit between the shaft hypotube 110 and the shaft rod 109 or by welding them together. The shaft hypotube 110 may in other embodiments of the invention be fixed directly into the barrel 118 either by a press-fit or by overmolding the barrel 118 onto the shaft hypotube 110. The proximal end of the balloon 102 is in this example bonded and sealed onto the outer diameter of the shaft proximal portion distal end 106 comprising the distal end of the shaft hypotube 110. The distal end of the balloon 102 is bonded and sealed onto the outer diameter of the most distal end of the shaft rod 109 at the distal shaft portion 107. Other relevant ways of providing a liquid passage along stiffening elements of a balloon catheter 102 for these procedures are widely available and well known to those skilled in the art and should be considered relevant alternative embodiments according to the invention.

The first embodiment presents unique advantages over prior art solutions. The integration of the inflation arrangement 115 and the balloon catheter 101 allows the physician to perform the procedure alone. Other prior art systems offer integration of the inflation arrangement and the balloon catheter into one system to be operated by one hand and by only the physician. However, the hand grip of the system may be a pistol grip, where the plunger of a syringe is pulled in a direction away from the balloon as a trigger. The grip of this and other embodiments of the invention has a conventional syringe-grip with a conventional syringe movement where a plunger is pushed by the thumb towards the balloon. All ENT physicians are used to handling a syringe from doing needle injections inside the nose, therefore a balloon dilation system offering the same grip and use is advantageous. The first embodiment and other embodiments of the invention are very simple and easy to understand, in that little or no assembly is needed and there are few moving parts. Other prior art solutions offer balloon dilations devices that have the balloon moving out from a balloon guiding tube and this increases the complexity of the system and also compromises the ergonomics since the thumb needs to push both the balloon forward and also push the plunger forward.

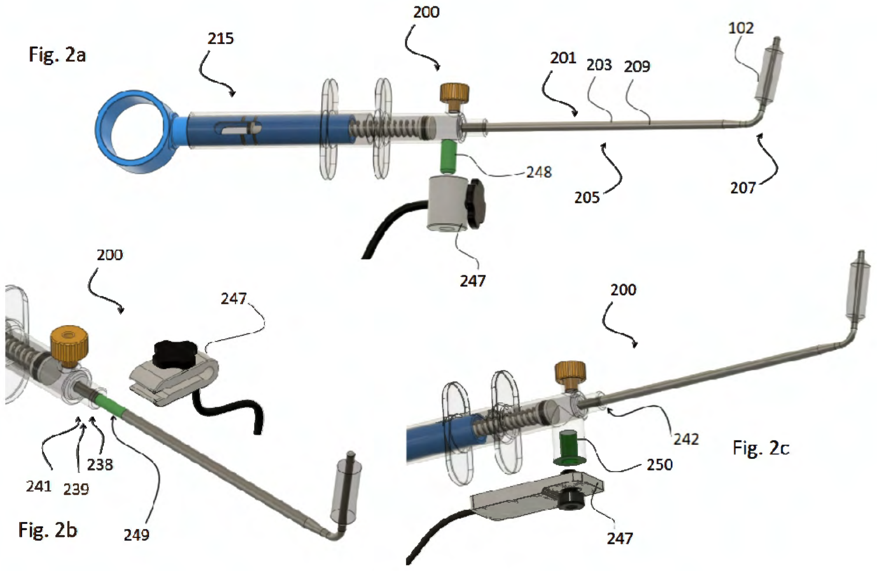

Figs. 2a-c show a second embodiment of the balloon inflation system 200 according to the invention wherein the inflation arrangement 215 is identical to the inflation arrangement 115 except for the features described below. The balloon inflation system shown in fig. 2a-c are all identical embodiments except for the features; sensor pin 248, sensor clamp mark 249 and sensor thread 250, that allows mounting of different types of a position sensor 247. The balloon inflation systems 200 are shown in a state where the balloon 202 is fully expanded and fully pressurized e.g. during a test before use, and where a position sensor 247 is aligned for being mounted. The balloon catheter 201 has a catheter shaft 203 comprising a one-piece shaft rod 209 with a distal shaft portion 207 that is bent to an 80-degree angle to be used for e.g. the frontal Sinus. The shaft rod 209 has a smaller diameter in the distal shaft portion 207 for ease of insertion into a passageway and a larger diameter at the proximal shaft portion 205 to provide a high stiffness. In another embodiments, the catheter shaft 203 comprises more than one part and may comprise two tubes with two diameters. It may be desired that the entire shaft rod 209 has a stiffness that prevents any part of the shaft rod 209 from deflecting more than e.g. 1mm during a balloon dilation procedure, as this would allow a position sensor 247 to be mounted on or near the syringe 216 to track the position of the balloon 202 with an accuracy of e.g. +/- 1 mm. The distal shaft portion 207 may be pre-bent. In other embodiments, other pre-bent angles and shapes will be available for other anatomical passageways. The distal end of the shaft rod 209 may be malleable, but with a stiffness high enough to avoid unintentional deflection during handling and during the procedure, and a stiffness high enough to require a bending tool for changing angle or shape. The shaft rod 209 has a shaft lumen 208 inside the shaft rod 209 with an open proximal end, a closed distal end and a side port inside the balloon 202 to fluidly connect the barrel volume 219 to the balloon 202. The proximal end of the shaft rod 209 is inserted directly into the syringe port 225 on the barrel 218 in a rigid connection portion 142 much like for the first embodiment of the balloon dilation system 100. In another embodiment according to the invention, the catheter shaft 203 may have a fluid connection tube on the outside of the shaft rod 209, either side by side with the shaft rod 209, or coaxially disposed around the shaft rod 209, and wherein the fluid connection tube fluidically connects the barrel volume 219 to the balloon 202. As seen in fig. 2a, a protruding sensor pin 248 on the syringe 216 may allow axially mounting of a position sensor 247 with a cylindrical hole and a fastening set-screw. The sensor pin 248 may in other examples be located on any part of the balloon inflation system 200 with a rigid connection directly or through one or more parts to the catheter shaft 203 except for the distal shaft portion 207. The sensor pin 248 may in other embodiments protrude directly from the proximal shaft portion 205, e.g. as an over-molded geometry or a metal rod or tube welded transversely onto part of the proximal shaft portion. As seen in fig. 2b, another embodiment has a sensor clamp marking 249 on the proximal shaft portion 205 to be used for mounting another position sensor 247 capable of being pinched transversely onto a shaft or a rib. According to another embodiment, the balloon dilation system 200 may have a sensor clamp marking 249 elsewhere on the balloon dilation system 200 for mounting of a pinching position sensor 247 onto another part of the system that is directly or through other parts rigidly connected to the catheter shaft 203. As seen in fig. 2c, a different embodiment of the invention may comprise a sensor thread 250 for mounting of a position sensor 247 onto the syringe 216 by means of a bolt or screw connection with a female thread as an integrated part of the syringe. In another embodiment, still according to the invention, the sensor mounting interfaces as described above may be mounted on any part of the balloon dilation system that has a rigid direct or indirect connection with the catheter shaft. The position sensors and mounting principles depicted in 2a-c are to be viewed as examples only. It may be relevant to use e.g. electromagnetic sensors that can be used in a system that generates an electromagnetic field or alternative visual sensors (not shown) that have three spheres or other visual markers visible to cameras of a visual navigation system. Both systems are widely used for tracking surgical equipment relative to the body of a patient after CT scan. Another embodiment of the invention has an electromagnetic sensor embedded directly into or permanently mounted onto a part of the inflation arrangement 215. In a preferred embodiment will an embedded or permanently mounted position sensor 247 be located on a reusable part of the balloon dilation system 200.

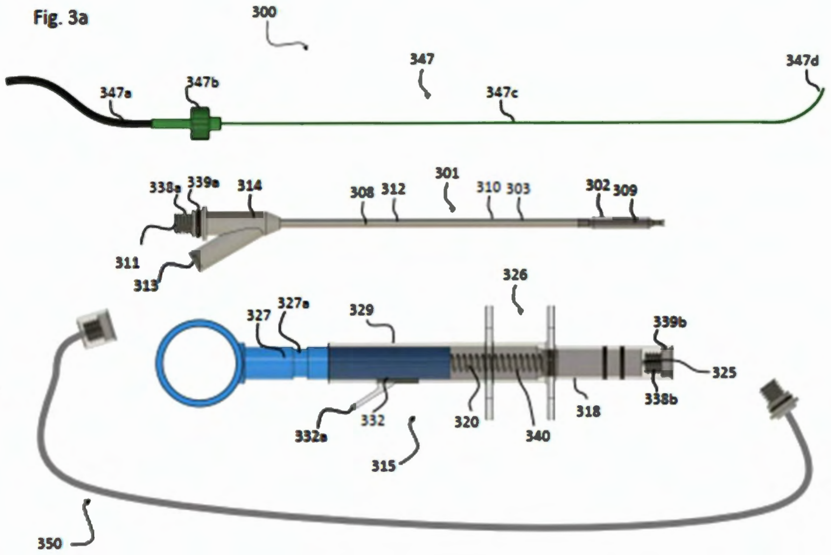

Figs. 3a-e show a third embodiment of a balloon dilation system 300 according to the invention in a disassembled and an assembled state.

Fig. 3a shows the third embodiment of a balloon dilation system 300 in a disassembled state.

A position sensor 347 has with a sensor plug 347b, a sensor cord 347a, a sensor shaft 347c and a sensor tip 347d.

An unattached balloon catheter 301 with a catheter shaft 303, a balloon 302, and a proximal catheter connector 314, the catheter connector 314 having the lumen port 311 and an access port 313 to a working channel 312 (not visible). The catheter shaft comprising a shaft hypotube 310 and a shaft rod 309 and wherein the balloon 302 is connected to the catheter shaft in the same way as seen in the balloon catheter 101 of the first embodiment. The shaft hypotube 310 and the shaft rod 309 are both bonded and integrated into the catheter connector 314 either by gluing or over molding. The catheter connecter 314 further having a male thread 338a to engage with a female thread 338b in the barrel 318 to form a mechanical coupling 338 and an O-ring 339a to engage with an O-ring seat 339b on the barrel 318, to form a fluid coupling 339. It is normal for syringes to have a male Luer Lock connection to engage with a female Luer Lock on e.g. needles to create a sufficient mechanical and fluid connection. It is normal to have a female Luer Lock on a balloon catheter for filling of a liquid, and a syringe with a male Luer Lock could in fact be connected directly to a balloon catheter without the conventional flexible connection tubes in between. The Luer lock coupling is, however, not suited for transferring the required bending moment from a stiff balloon catheter shaft used for e.g. insertion into the nose of a person. It is very normal that the physician wishes to push tissue inside the nose in a direction transverse to the insertion direction and transverse to a catheter shaft and this demands for a catheter shaft to have a certain stiffness and for the coupling from a catheter shaft to a handle to have a certain robustness and stiffness. This is one of the reasons why most commercially available balloon insertion instruments to be used in the nose of a patient have an instrument handle mechanically coupled directly to catheter shaft to be able to transfer forces from the hand of the physician to a catheter shaft and with a flexible tube between a balloon catheter Luer Lock and a syringe Luer Lock to protect the Luer Lock coupling from any forces that could result in a leakage. If a physician were to grasp a conventional syringe to control and move a stiff balloon catheter shaft inside the nose of a person, via a direct Luer Lock coupling, then it is very likely that the coupling would break and/or leak.

One way of making a sufficiently robust coupling between a syringe and a balloon catheter shaft is with a glued connection as seen in the first and second embodiments. However, this is a permanent connection. It may be desirable to be able to disassemble the balloon catheter 301 from the syringe 318 if it is needed to switch to another embodiment of the syringe 318 or another embodiment of the balloon catheter 301 e.g. an embodiment that is a balloon inflation system kit according to the invention comprising two or more balloon catheters 301 suitable for different passageways. It may also be desired to fill the barrel 318 from the syringe port 325.

The working channel 312 extends from the access port 313 through the catheter connector 314, through the shaft rod 309, to an opening at the most distal end of the shaft rod 309 and distal to the balloon 302. The balloon dilation system 300 may be filled with liquid and assembled after unpacking by an operator or it may pre-filled and preassembled prior to packaging at a manufacturing site.

An inflation arrangement 315 similar to the first and second embodiments of the balloon dilation system 100 and 200, with the same or similar internal interfaces between the plunger 320, the spring 340 and the thruster 327, but in this particular embodiment with a thruster lock 332 for locking the thruster 327 relative to the thruster housing 329 at an axial end stop position. The thruster lock 332 is in this example a molded part of the thruster housing 329 and is predisposed to move radially inwards and lock against a radially in-cut groove 327a in the thruster 327, when axially aligned. The thruster lock 332 has a lock lever 332a for releasing the thruster lock 332. The thruster 327 is releasable lockable relative to the barrel 318 via the thruster housing 329. The thruster lock 332 may in other embodiments be a different type of releasable lock and may be connected to different parts of the system in order to lock the thruster 327 in a specific axial position relative to the barrel 318. The spring 340 is in a first pre-compressed length. The thruster 327 and the plunger 320 are fully retracted. A flexible fluid connection tube 350 may optionally be used to connect the balloon catheter and the inflation arrangement or the balloon catheter may be mounted directly on the inflation arrangement. The connection by the fluid connection tube may allow an operator increased flexibility.

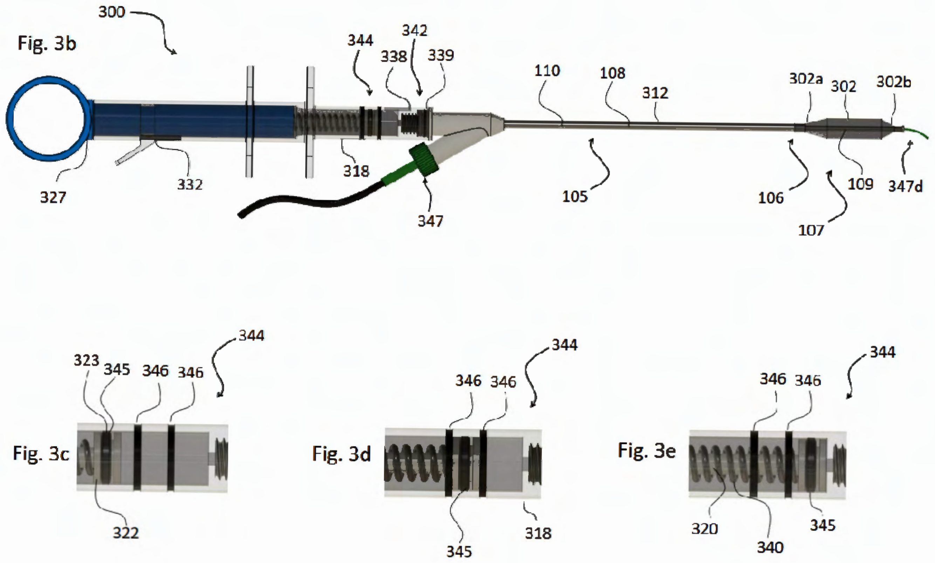

Fig. 3b shows the third embodiment of the balloon dilation system 300 in an assembled state, wherein the balloon catheter 301 is connected with the inflation arrangement 315 by the threads 338a and 338b, seen in fig. 3a, to form a mechanical coupling 338 and a rigid connection portion 342 and wherein O-ring 339a seals against the O-ring surface 339b, seen in fig 3a, to form a fluid coupling 339. The balloon 302 is expanded and pressurized, the thruster 327 has been moved a first distance distally to an end stop position, where it is automatically locked by the thruster lock 332, and the plunger 320 has moved a second distance distally into the barrel 318, wherein the first distance is longer than the second distance. The spring 340 has been compressed from its first pre-compressed length, to a second shorter compressed length. The difference between the travelled distance of the thruster 327 and the plunger 320 corresponds to the reduction in the length of the spring 340. The pressure of the system is within a predefined setpoint pressure range (such as a selected range) of e.g. 12 bar +/- 1 bar. The plunger head seal 323 comprises a first pressure dial 345 of a pressure indicator 344. Two marked lines on the barrel 318 comprise a second pressure dial 346 of a pressure indicator 344. The plunger head seal 223 and thereby the first pressure dial 345 is aligned axially between the two lines on the barrel 318 that comprises the second pressure dial 346, the pressure indicator 344 thereby indicating that the pressure is within the setpoint pressure range (such as a selected range). This state of the pressure indicator 344 can also be seen in detail in fig. 3d. If the first pressure dial 345 is distal from any part of the second pressure dial 346 in this state, as can be seen in fig. 3e, then the pressure is below the setpoint pressure range, which may be caused by leakage or insufficient filling of liquid into the barrel 318. If the first pressure dial 345 is proximal from any part of the second pressure dial 346 in this state, as can be seen in fig. 3c, then the pressure is above the setpoint pressure range. The pressure may still be within an acceptable range for the treatment and be below the balloon burst pressure. Other embodiments, still according to this invention may include other ways of viewing a change in the relative position between a part of a plunger and a part of a barrel. In this embodiment, the thruster 327 may be pushed to the end-stop position without carefully monitoring the pressure indicator 344. The spring 340 is designed with a very low spring rate such that any additional compression of the spring 340 only causes an insignificantly increased force and where the increased hydrostatic internal pressure as a result of additional spring compression is below the burst pressure of the balloon and within an acceptable pressure range approved for the procedure. The spring rate of the spring 340, the barrel volume 319, the internal volumes balloon catheter 301 and the stroke length of the thruster 327 is designed to ensure that the internal hydrostatic pressure never exceeds the balloon burst pressure, even when the thruster 327 is pushed to the end stop and even when the balloon 302 is restricted from expanding to its fully expanded diameter. A functionality like this is important for an inflation arrangement 315 when held by the operator e.g. a physician who also inserts the balloon catheter 301 with the same hand, because the operator needs to focus on viewing the patient anatomy and balloon placement. Normally, the pressurization is done by an assistant.

Other inflation devices exist where there are features to automatically control the pressure and without a need for monitoring pressure gauges or pressure indicators during movement of the plunger. One device has a pressure shut off valve that blocks the liquid passage between the syringe and the balloon when the pressure setpoint (such as a selected range) is reached. Another device has a locking mechanism for locking the plunger head relative to the barrel in any position when the pressure setpoint (such as a threshold pressure) is reached. If a balloon of the prior art systems is not fully inflated immediately due to restrictions in the anatomy of the patient despite the pressure being at or within the setpoint, then the system is locked in that position and there is no feedback to the operator that the balloon is not fully inflated. Also, if the tissue around a balloon of such a prior art system slowly yields to the pressure from said balloon, then these systems will lose the pressure in their locked state and since their plunger is not spring-loaded and free to move in the locked position, then the balloon of these systems will not be able to automatically push further onto to the tissue while it yields, to provide the desired dilation corresponding the to the fully expanded diameter of the balloon. These prior art systems also cannot show a loss in pressure after the system has been locked. And so, the physician will receive no information about neither pressure loss nor insufficient balloon expansion during the attempt to dilate the anatomy. This may lead to unrecognized non-treatment of the patient, since the operator trusts that the locked or depressed state is sufficient, without being able to see the state of the balloon, which is hidden inside the passageway and only partly visible. It would be advantageous to have an inflation system that provides information about pressure loss without a conventional pressure gauge. It would be advantageous to have an inflation system that has a spring-loaded plunger to keep the desired setpoint pressure in a locked position, even when the balloon slowly reaches its fully expanded diameter due to resistance in the tissue or bone structure in the passageway of the patient.

This is all achieved with the design of the spring 340 of this third embodiment. Since the thruster 327 is locked to the barrel 318 in the locked position and since the plunger 320 is spring-loaded and free to move relative to the locked thruster 327, then the spring-loaded plunger 320 will hold an acceptable pressure in the barrel volume 319 and thereby in the balloon 302, even if the balloon 302 changes from being 20% expanded, to being 100% expanded, and so, the balloon 302 will keep pushing onto the tissue in the passageway until it reaches the maximum diameter of the balloon 302, if the tissue allows it. The spring 340 has a very low spring rate to make sure that changes in position of the plunger 320 relative to the locked thruster 327 results in insignificant changes in forces from the spring 340 and thereby insignificant pressure changes. The spring 340 may in this embodiment be designed so that the plunger in any possible position, when the thruster 327 is in the locked position, provides a pressure in the barrel volume 319 that is below the burst pressure of the balloon 302 and above the required pressure to perform the dilation procedure.

The spring 340 with a very low spring rate, needs to have a very long free length to be able to deliver the required spring force at a given spring compression length. For the sake of ergonomics, it is desired to have a short stroke of the thumb of the hand to fill and pressurize the balloon 302 and a long spring 340 with a low spring rate would therefore need to be held in a pre-compressed state between the thruster 327 and the plunger 320 with a pre-compressed length corresponding to or slightly below the spring-force needed to create a hydrostatic pressure in the barrel volume 319 by means of the plunger 320. A way of describing the optimal spring 340 may be by the ratio between the pre-compressed length and the free length of the spring 340. The spring 340 is preferably pre-compressed to a length shorter than 80% of its free length, more preferred shorter than 70% or even more preferred shorter than 60%, or optimally shorter than 50% of its free length. In fig. 3b, the position sensor 347 is shown in a fully inserted and locked position, where the sensor tip 347d extends out from the distal end of the balloon 302. The sensor 347 may comprise an electromagnetic sensor, with the sensor coil being located at the sensor tip 347d. In other embodiments, the sensor tip 347d may be positioned between a distal balloon end 302a and a proximal balloon end 302b. Other navigation elements, such as a guidewire or a light fiber may be inserted through the working channel 312 to help locate the correct anatomical passageway. The access port 313 may be connected to a water filled syringe for pumping of water or medicine to the area of treatment through the working channel 312. The access port 313 may be connected to a source of vacuum to facilitate suction at the area of treatment through the working channel 312. Other purposes of the working channel 312 may be relevant in other embodiments. The various ways of uses of the working channel 312 in the balloon catheter 301 for this procedure are well known to people skilled in the art.

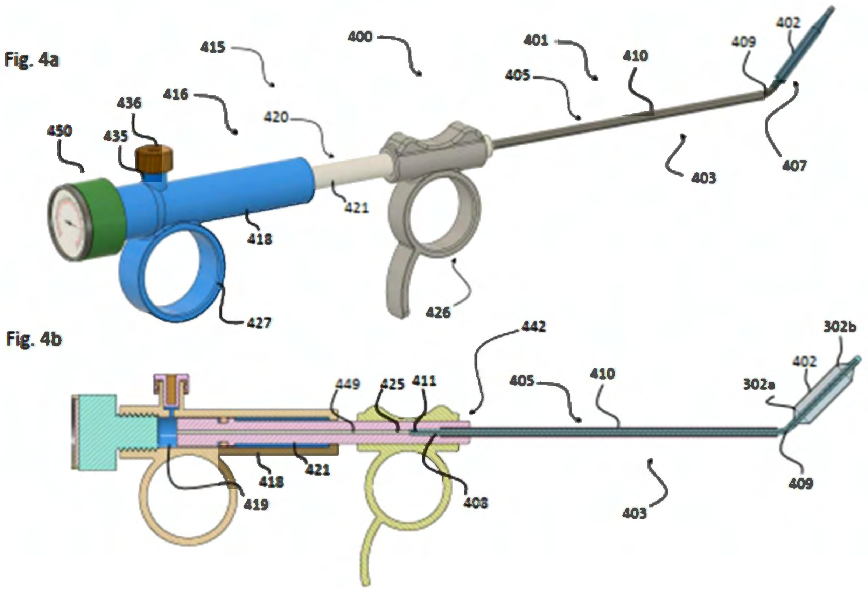

Fig. 4a and 4b shows a fourth example of the balloon dilation system 400 in perspective view and in section view. A syringe 416 is in a reversed configuration with a thruster 427 with a thumb ring being part of the barrel 418 and the grasping portion 426 with a finger ring being attached to the plunger 420. The barrel 418 has a filling port 435 for filling liquid into the barrel 418 and a filling port plug 436 for sealing the filling port 435 after filling. A pressure gauge 450 is mounted into a threaded hole in the proximal end of the barrel 418 and in fluid connection with the barrel volume 419 to measure and show the pressure inside the barrel volume 419. It may be favorable to have the pressure gauge 450 in the most proximal end of the system to have it visible at all times during insertion of the balloon catheter 401 and handling of the system. The plunger rod 421 has a plunger lumen 449 for fluidically connecting the barrel volume 419 with the shaft lumen 408. The distal end of the plunger rod 421 has a syringe port 425 in fluid connection with the lumen port 408 of a balloon catheter 401.

The shaft 403 of the balloon catheter 401 has an inner shaft rod 409 with an internal shaft lumen 408 and a proximal lumen port 411. A shaft rod 109 is glued directly into the syringe port 425 inside the distal end of the plunger rod 421 to create a fluid connection between the barrel 418 and the shaft lumen 408. The shaft rod 409 is closed in the distal end tip and has a fluid opening between the proximal ballon end 402a and the distal balloon end 402b to fluidly connect the ballon 402 to the shaft lumen 408. Both the proximal balloon end 402a and the distal balloon end 402b are bonded and sealed onto the distal end of the shaft rod 409. The shaft rod 409 is shown in a bent position where the bend is located proximal to the proximal balloon end 402a. The bend or curved section of the distal shaft rod 409 may alternatively be partially or entirely placed between the balloon proximal end 402a and the balloon distal end 402b. The proximal shaft portion 405 comprises a shaft hypotube 410 to provide the required stiffness to the catheter shaft 403 and has a proximal end that is glued directly into a hole in the distal end of the plunger rod 421. The proximal shaft portion 405 is secured into the distal end of the plunger rod 421 in a rigid connection portion. Also, according to the invention, other ways of creating a fluid path from the syringe barrel volume 419 through the plunger 420 and through the catheter shaft 403 may include flexible polymer tubing in combination with steel tubes that provide stiffness.

Fig. 4a shows the ballon dilation system 400 in a first state, where the barrel 418 is in a retracted proximal position and where the balloon 402 is deflated. Fig. 4b shows the balloon dilation system 400 in a second state, where the barrel 418 is pushed forward relative to the plunger 420 and where the balloon is in an inflated state.

In the following clauses desired aspects and embodiments of the invention are specified:
Clause 1. A balloon dilation system suitable for balloon dilation of an anatomic passageway, the balloon dilation system comprising:
   a balloon catheter comprising;
      a catheter shaft having a shaft length, a shaft axis, a proximal shaft portion, a distal shaft portion and a shaft lumen,
      an inflatable balloon located at the distal shaft portion, the inflatable balloon having a proximal balloon end and a distal balloon end and the inflatable balloon being in fluid connection with the shaft lumen, and
      a lumen port at the proximal shaft portion in fluid connection with the shaft lumen,
   an inflation arrangement comprising;
      a syringe comprising a barrel having a barrel volume, a syringe axis, and a plunger with a plunger rod and a plunger head,
      a syringe port in fluid connection with the barrel volume,
      a grasping portion engaged with, connected to or forming part of one of the plunger and the barrel,
      a thruster engaged with, connected to or forming part of another one of the plunger and the barrel,
   wherein the barrel volume is in fluid connection with the shaft lumen,
   wherein the balloon dilation system comprises a rigid connection portion,
   wherein the balloon catheter and the inflation arrangement are coupled to or are integrated with each other in said rigid connection portion and
   wherein the rigid connection portion is in a fully rigid connection portion wherein all motions of the entire inflation arrangement are translated to the balloon catheter.
Clause 2. The balloon dilation system according to clause 1, wherein the proximal shaft portion extends at least 50 mm from the rigid connection portion and wherein the proximal shaft portion determined in a length section L extending from the rigid connection portion to 50 mm or more from the rigid connection portion has a flexural stiffness of at least 1000 Nmm², preferably the length section L extending from the rigid connection portion to 50 mm or more from the rigid connection portion has a flexural stiffness of at least 5000 Nmm², such as at least 10000 Nmm², such as at least 25000 Nmm², such as at least 50000 Nmm², such as at least 75000 Nmm², such as at least 80000 Nmm², preferably determined by the three-point-bending test method and/or by the single sided clamp method.
Clause 3. The balloon dilation system according to clause 1 or clause 2, wherein the proximal shaft portion extends at least 70 mm from the rigid connection portion and wherein the proximal shaft portion has a flexural strength determined in a length section L extending from the rigid connection portion to 70 mm or more from the rigid connection portion which is at least 1000 Nmm², preferably the length section L extending from the rigid connection portion to 70 mm or more from the rigid connection portion has a flexural stiffness of at least 5000 Nmm², such as at least 10000 Nmm², such as at least 25000 Nmm², such as at least 50000 Nmm², such as at least 75000 Nmm², such as at least 80000 Nmm², preferably determined by the three-point-bending test method and/or by the single sided clamp method.
Clause 4. The balloon dilation system according to any one of the preceding clauses, wherein the proximal shaft portion determined in a length section L extending from the rigid connection and in the entire proximal shaft portion has a flexural stiffness of at least 1000 Nmm², preferably the length section L extending from the rigid connection and in the entire proximal shaft portion has a flexural stiffness of at least 5000 Nmm², such as at least 10000 Nmm², such as at least 25000 Nmm², such as at least 50000 Nmm², such as at least 75000 Nmm², such as at least 80000 Nmm², preferably determined by the three-point-bending test method and/or by the single sided clamp method.
Clause 5. The balloon dilation system according to any one of the preceding clauses, wherein the proximal shaft portion has a stiffness determined using the single sided clamp method, wherein the clamp location is adjacent a proximal shaft end of the proximal shaft portion and the force applying location is adjacent the proximal shaft portion distal end and wherein the proximal shaft portion has a stiffness sufficiently high to prevent the proximal shaft portion distal end from deflecting more than 20 mm when a force of 1N is applied to the proximal shaft portion at a location adjacent to the proximal shaft portion distal end in a direction transverse to the shaft axis, preferably the proximal shaft portion distal end has a deflection not exceeding 18 mm, such as not exceeding 16 mm, such as not exceeding 14 mm when a force of 1N is applied to the proximal shaft portion at a location adjacent to the proximal shaft portion distal end in a direction transverse to the shaft axis.
Clause 6. The balloon dilation system according to any one of the preceding clauses, wherein the proximal shaft portion has a force-to-deflection ratio k determined using the single sided clamp method, wherein the clamp location is adjacent a proximal shaft end of the proximal shaft portion and the force applying location is adjacent the proximal shaft portion distal end and wherein the force-to-deflection ratio k is at least 0.1 N/mm, such as at least 0.2 N/mm, such as, at least 0.3 N/mm, such as, at least 0.4 N/mm, such as at least 0.5 N/mm, such as at least 0.6 N/mm, such as at least 0.7 N/mm, such as at least 0.8 N/mm.
Clause 7. The balloon dilation system according to any one of the preceding clauses, wherein the rigid connection portion interconnects said balloon catheter and said inflation arrangement and wherein the rigid connection portion has a flexural stiffness larger than or identical to the flexural stiffness of at least a length section L extending from the rigid connection portion to 50 mm or more from the rigid connection portion, preferably the rigid connection portion has a flexural stiffness which is at least 105%, such as at least 110%, such as at least 120% of the flexural stiffness of at least the length section L extending from the rigid connection portion to 50 mm or more from the rigid connection portion.
Clause 8. The balloon dilation system according to any one of the preceding clauses, wherein the proximal shaft portion comprises or consists of a length portion of the catheter shaft comprising an annular metallic reinforcement layer, such as a shaft hypotube, an externally located steel tube and/or an annular reinforcement.
Clause 9. The balloon dilation system according to any one of the preceding clauses, wherein the balloon dilation system comprises a fluid connection between said syringe port and said lumen port, wherein said fluid connection optionally comprises a valve arrangement for closing and opening said fluid connection.
Clause 10. The balloon dilation system according to clause 9, wherein said fluid connection between said barrel volume and said shaft lumen is provided by a fluid coupling between said syringe port and said lumen port.
Clause 11. The balloon dilation system according to clause 10, wherein the fluid coupling constitutes or forms part of the rigid connection portion, wherein the fluid coupling optionally comprises an annular reinforcement, such as a metallic reinforcement ring.
Clause 12. The balloon dilation system according to any one of the preceding clauses, wherein the rigid connection portion at least in a first mode of said balloon dilation system is a linear rigid connection portion allowing rotational motions of said balloon catheter and said inflation arrangement relative to each other.
Clause 13. The balloon dilation system according to any one of the preceding clauses, wherein the grasping portion is located with a first distance d1 to the proximal balloon end and the thruster is located with a second distance d2 to the proximal balloon end and wherein the second distance d2 is larger than the first distance d1; and/or
   wherein the inflation arrangement has a distal end defined by the syringe port and wherein the grasping portion is located distally to the thruster.
Clause 14. The balloon dilation system according to any one of the preceding clauses, wherein the rigid connection portion comprises a coupling, such as a mechanical coupling, preferably said mechanical coupling comprises said fluid coupling, optionally said mechanical coupling is a separable mechanically coupling.
Clause 15. The balloon dilation system according to any one of the preceding clauses, wherein the rigid connection portion comprises a glued coupling or a partly mechanical-partly glued coupling, wherein the rigid connection portion comprises a bonding material, such as an UV cross-linked polymer.
Clause 16. The balloon dilation system according to any one of the preceding clauses 1-13, wherein the rigid connection portion comprises an integration portion integrating said balloon catheter and said inflation arrangement with each other, preferably to provide that the balloon catheter and the inflation arrangement form one unified structure.
Clause 17. The balloon dilation system according to clause 16, wherein the integration portion comprises at least an integration between said syringe port and said lumen port, preferably to ensure the fluid connection between the barrel volume and the shaft lumen.
Clause 18. The balloon dilation system according to any one of the preceding clauses, wherein the inflatable balloon is coaxially disposed about the distal shaft portion, preferably the distal shaft portion extends distally beyond the distal balloon end wherein the inflatable balloon is connected to the distal shaft portion at or adjacent to the distal balloon end.
Clause 19. The balloon dilation system according to any one of the preceding clauses, wherein the balloon dilation system is adapted for being in a first balloon dilation system state, wherein the balloon is non-expanded, the plunger is in a retracted position, and the thruster is in a first thruster position, and in a second balloon dilation system state wherein the balloon is expanded, the plunger is in a deployed position, and the thruster is in a second thruster position.
Clause 20. The balloon dilation system according to clause 19, wherein the second balloon dilation system state comprises a fully activated balloon dilation system state wherein the balloon is fully expanded, the plunger is in a fully deployed position, and the thruster is in a fully activated position and a transition balloon dilation system state wherein the balloon dilation system is in a state between the first balloon dilation system state and the fully activated balloon dilation system state.
Clause 21. The balloon dilation system according to any one of the preceding clauses, wherein the barrel volume comprises a liquid and/or wherein the inflation arrangement comprises a closable liquid filling port, suitable for filling liquid into the barrel volume, optionally the liquid filling port form part of or is constituted by the syringe port.
Clause 22. The balloon dilation system according to any one of the preceding clauses, wherein the rigid connection portion, such as the mechanical coupling, the mechanical-glued coupling or the integration portion has a rigidity that is sufficiently high to prevent at least the proximal shaft portion of the catheter shaft, such as the entire catheter shaft, from rotating and translating relative to the inflation arrangement, preferably the rigid connection portion is capable of withstanding an axial displacement force of at least 1N, such as at least 10 N and/or a torsional stiffness of at least 10 N/mm, preferably at least 20 N/mm.
Clause 23. The balloon dilation system according to any one of the preceding clauses, wherein the rigid connection portion, such as the mechanical coupling, the mechanical-glued coupling or the integration portion has a rigidity that is sufficiently high to provide that the rigid connection portion will be permanently damaged, such as fully or partially break before it will bend.
Clause 24. The balloon dilation system according to any one of the preceding clauses, wherein the rigid connection portion is sufficiently strong to withstand a bending moment from the catheter shaft of at least 50 N/mm, such as a bending moment of at least 60 N/mm, such as a bending moment of at least 75 N/mm or higher.
Clause 25. The balloon dilation system according to any one of the preceding clauses, wherein the proximal shaft portion extends from the rigid connection portion and to a proximal shaft portion distal end, wherein the proximal shaft portion distal end is located proximally to the distal balloon end, preferably the proximal shaft portion distal end is located proximally to a mid-length location of the inflatable balloon, wherein the mid-length location of the inflatable balloon is a middle location between the proximal balloon end and the distal balloon end.
Clause 26. The balloon dilation system according to any one of the preceding clauses, wherein the proximal shaft portion extends from the rigid connection portion and to a proximal shaft portion distal end, wherein the proximal shaft portion distal end is located proximally to the balloon, and preferably with a distance to the balloon of up to 50% of the shaft length, such as up to 20% of the shaft length.
Clause 27. The balloon dilation system according to any one of the preceding clauses, wherein the proximal shaft portion extends from the rigid connection portion and to a proximal shaft portion distal end located at least 70 mm from the rigid connection portion.
Clause 28. The balloon dilation system according to any one of the preceding clauses, wherein the proximal shaft portion extends from the rigid connection portion and to a proximal shaft portion distal end located at least 90 mm from the rigid connection portion.
Clause 29. The balloon dilation system according to any one of the preceding clauses, wherein the thruster is displaceable, preferably axially displaceable relative to the grasping portion.
Clause 30. The balloon dilation system according to any one of the preceding clauses, wherein the inflation arrangement comprises at least one linear thruster track and wherein the thruster comprises at least one track grip adapted for engage with and slide along the at least one thruster track, wherein the at least one linear thruster track is parallel with the syringe axis.
Clause 31. The balloon dilation system according to any one of the preceding clauses, wherein the thruster is partially and displaceable located in a thruster housing, wherein the thruster housing optionally is integrated with or coupled to the barrel, preferably integrated with or coupled to a proximal end portion of the barrel.
Clause 32. The balloon dilation system according to clause 31, wherein the thruster has a distal thruster housing passage into the barrel, wherein the plunger rod is displaceable passing through the distal thruster housing passage, wherein the plunger rod has a protuberance located in the thruster housing to prevent the plunger from slipping out of the thruster housing.
Clause 33. The balloon dilation system according to any one of the preceding clauses, wherein said grasping portion protrudes outwards from an outer annular wall face of said barrel and/or outwards from an outer wall face said thruster housing, optionally said grasping portion protrudes radially or with an angle to the syringe axis of at least 45° from the outer annular wall face of said barrel and/or outwards from the outer wall face said thruster housing.
Clause 34. The balloon dilation system according to any one of clauses 31-33, wherein at least a head portion of the thruster is located outside the thruster housing, preferably at least a head portion of the thruster is located outside the housing even when the thruster is in the second thruster position.
Clause 35. The balloon dilation system according to any one of clauses 31-34, wherein the thruster housing comprises a thruster guide section, comprising the at least one linear thruster track.
Clause 36. The balloon dilation system according to any one of clauses 31-35, wherein the thruster housing comprises a thruster lock adapted for temporally locking the thruster in the second thruster position.
Clause 37. The balloon dilation system according to any one of the preceding clauses, wherein the thruster is engaged with the plunger or the barrel via a gearing arrangement, wherein the gearing arrangement is arranged to reduce the force required to deploy the thruster from the first thruster position to the second thruster position, preferably the gearing arrangement is engaged with the thruster and the plunger head to provide that the thruster is pivotally movable relative to the plunger.
Clause 38. The balloon dilation system according to any one of the preceding clauses, wherein the inflation arrangement comprises an inflation arrangement housing and optionally the grasping portion is located at or form part of the inflation arrangement housing,
Clause 39. The balloon dilation system according to clause 38, wherein the thruster is pivotally engaged with or connected to the plunger or the barrel, wherein a pivoting motion of the thruster comprises pivoting the thruster relative to said inflation arrangement housing, whereby the thruster effectuates a motion of one of the plunger and the barrel.
Clause 40. The balloon dilation system according to clause 38 or clause 39, wherein the thruster is partially and displaceable located in a thruster housing, wherein the thruster housing optionally is integrated with or coupled to the barrel, preferably integrated with or coupled to a proximal end of the barrel.
Clause 41. The balloon dilation system according to any one of the preceding clauses 38-40 clauses, wherein the fluid connection between the barrel volume and the catheter shaft lumen comprises an intermediate flexible tube section, located inside or outside said inflation arrangement housing.
Clause 42. The balloon dilation system according to any one of the preceding clauses, wherein the thruster and the grasping portion are movable relative to each other via a threaded engagement to move the plunger and the barrel relative to each other.
Clause 43. The balloon dilation system according to any one of the preceding clauses, wherein the thruster comprises a thumb ring, the thruster ring is preferably adapted to be pushed and optionally pulled by a thumb of an operator.
Clause 44. The balloon dilation system according to any one of the preceding clauses, wherein the thruster forms part of or is constituted by the plunger.
Clause 45. The balloon dilation system according to any one of the preceding clauses, wherein the grasping portion is connected to or integrated with the barrel, and wherein the thruster is operably coupled to the plunger rod and/or the plunger head.
Clause 46. The balloon dilation system according to any one of the preceding clauses, wherein the syringe port forms part of the barrel and wherein the syringe port is directly connected to the lumen port, preferably without any other fluid connection parts in between.
Clause 47. The balloon dilation system according to any one of the preceding clauses, wherein the rigid connection portion directly interconnects the barrel and the catheter shaft, preferably the rigid connection portion comprises a mechanical coupling and wherein the mechanical coupling is establishing a rigid coupling directly between the barrel and the catheter shaft.
Clause 48. The balloon dilation system according to any one of the preceding clauses, wherein the rigid connection portion directly interconnects the barrel and the catheter shaft.
Clause 49. The balloon dilation system according to any one of the preceding clauses, wherein the syringe axis and the catheter shaft axis are parallel.
Clause 50. The balloon dilation system according to any one of the preceding clauses, wherein the syringe axis and the catheter shaft axis have an angle to each other of from 0 to 30°, such as from 0 to 20°
Clause 51. The balloon dilation system according to any one of the preceding clauses, wherein the syringe is an inverted syringe, wherein the grasping portion is engaged with, connected to or form part of the plunger rod and wherein the thruster is operably coupled to the barrel for pushing the barrel over the plunger rod in a distal direction towards the balloon catheter to inflate the balloon.
Clause 52. The balloon dilation system according to clause 51, wherein the plunger rod comprises a plunger lumen fluidically connecting barrel volume with syringe port.
Clause 53. The balloon dilation system according to clause 51, wherein the rigid connection portion directly interconnects the plunger rod and the catheter shaft, preferably the rigid connection portion comprises a mechanical coupling and wherein the mechanical coupling is establishing or forming part of a rigid coupling directly between the plunger rod and the catheter shaft.
Clause 54. The balloon dilation system according to any one of the preceding clauses, wherein the catheter shaft has a distal end and comprises a shaft rod that extends from the rigid connection portion and along the entire catheter shaft length to the distal end of the catheter shaft, wherein the shaft rod preferably forms an internal part of the catheter shaft.55. The balloon dilation system according to any one of the preceding clauses, wherein the shaft lumen in at least a length portion of the catheter shaft is in the form of a gap between an outer surface of the shaft rod and an inner surface of a fluid connection tube disposed concentrically about the rod.
Clause 56. The balloon dilation system according to any one of the preceding clauses 1-54, wherein the shaft lumen in at least a length portion of the catheter shaft is in the form of a channel inside the shaft rod.
Clause 57. The balloon dilation system according to any one of the preceding clauses 54-56, wherein the shaft rod has a distal end with a curved portion located at said distal shaft portion and proximally to the distal balloon end, wherein the distal shaft portion preferably extends from the distal end of the catheter shaft and at least to a mid-length location of the inflatable balloon, wherein the mid-length location of the inflatable balloon is a middle location between the proximal balloon end and the distal balloon end.
Clause 58. The balloon dilation system according to clause 57, wherein the distal shaft portion extends from the distal end of the catheter shaft and at least to the proximal balloon end, such as to 1 cm distal to the proximal balloon end, such as to a distal end of a hypotube.
Clause 59. The balloon dilation system according to any one of clauses 54-58 wherein the entire shaft rod has a stiffness that prevents elastic and/or plastic deformation of more than 2 mm in any direction and at any point of the catheter shaft during a normal procedure involving an axial force on the shaft rod not exceeding 10 N, and a transverse force at any point of the shaft rod not exceeding 1N.
Clause 60. The balloon dilation system according to any one of clauses 54-59 wherein the shaft rod has a distal end and a stiffness sufficiently high to prevent the distal end of the shaft rod from deflecting beyond 5 mm, preferably 1 mm or less when a applying a force F at the distal end of the shaft rod, wherein F is up to 1 N, e.g. using the single sided clamp method.
Clause 61. The balloon dilation system according to any one of the preceding clauses 54-60, wherein the shaft rod has a distal end, and wherein the distal end of the shaft rod is malleable and preferably adapted for being shaped by an operator.
Clause 62. The balloon dilation system according to any one of the preceding clauses, wherein at least a part of the proximal shaft portion comprises a shaft hypotube and wherein the shaft hypotube preferably is located to form an annular reinforcement of at least the part of the proximal shaft portion.
Clause 63. The balloon dilation system according to clause 62, wherein the shaft lumen at least in the distal shaft portion is located between an outer surface of the shaft rod and an inner surface of the shaft hypotube and wherein the proximal end of the balloon is bonded to the distal end of the shaft hypotube and wherein the distal end of the balloon is bonded to a distal end of the shaft rod.
Clause 64. The balloon dilation system according to any one of the preceding clauses, wherein the balloon catheter comprises a working lumen suitably arranged for insertion of at least one of a guide wire, a light fiber, an electromagnetic sensor or for delivering a liquid substance, or to facilitate suction, and wherein the balloon catheter comprises an access port to the working channel
Clause 65. The balloon dilation system according to clause 64, wherein at least a distal length section of the working lumen is located inside and extends lengthwise in the shaft rod.
Clause 66. The balloon dilation system according to clause 64 or clause 65, wherein the access port to the working lumen is located at and forms a proximal end of the working lumen and wherein at least a distal length section of the working lumen is coaxial with the shaft axis.
Clause 67. The balloon dilation system according to clause 64 or clause 65, wherein the access port to the working channel is located distally to the lumen port at an acute angle relative to the catheter center axis.
Clause 68. The balloon dilation system according to any one of clauses 64-67, wherein the balloon catheter comprises a catheter connector arranged for connecting the inflation arrangement and the balloon catheter and forming part of the rigid connection portion, optionally the catheter connector comprises a connector working lumen forming part of the working lumen with a proximal access port into the connector working lumen, preferably the catheter shaft comprises a shaft working lumen forming part of the working lumen and located to form an extension of the connector working lumen.
Clause 69. The balloon dilation system according to clause 68, wherein at least a part of the catheter connector forms part of or constitutes the rigid connection portion, preferably at least the catheter shaft branch and the distal catheter connector branch form part of or constitutes the rigid connection portion.
Clause 70. The balloon dilation system according to clause 68 or clause 69, wherein the lumen port is located in the catheter connector and preferably comprises a fluid passage orthogonal to the shaft axis.
Clause 71. The balloon dilation system according to any one of the preceding clauses 1-69, wherein the lumen port is located at a proximal end of the catheter shaft and has a lumen port length coaxial with the shaft axis.
Clause 72. The balloon dilation system according to any one of the preceding clauses, wherein the syringe port extends transverse to the syringe axis across a wall of the syringe.
Clause 73. The balloon dilation system according to any one of the preceding clauses 1-71, wherein the syringe port extends parallel to the syringe axis and forms a distal tip of the syringe and wherein the syringe is connected directly to the catheter shaft.
Clause 74. The balloon dilation system according to any one of the preceding clauses, wherein the proximal shaft portion comprises a visually marked area adapted for clamping on a clamp-type sensor or wherein the proximal shaft portion comprises a clamp-type sensor clamped on to the proximal shaft portion, wherein the clamp-type sensor is electrically connected to or is electrically connectable to an electromagnetic or visual navigation system.
Clause 75. The balloon dilation system according to any one of the preceding clauses, wherein at least a length section of the proximal shaft portion comprises an externally located steel tube, preferably having a diameter of at least 3 mm, a length of at least 10 mm and a wall thickness of at least 0.2 mm.
Clause 76. The balloon dilation system according to clause 75, wherein the steel tube comprises or constitutes a visually marked area adapted for clamping on a clamp-type sensor or wherein the proximal shaft portion comprises a clamp-type sensor clamped on to the proximal shaft portion, wherein the clamp-type sensor is electrically connected to or is electrically connectable to an electromagnetic or visual navigation system.
Clause 77. The balloon dilation system according to any one of the preceding clauses, wherein the inflation arrangement comprises a protruding cylindrical pin, wherein the protruding cylindrical pin is adapted for clamping on a clamp-type sensor or wherein the protruding cylindrical pin comprises a clamp-type sensor clamped on to the protruding cylindrical pin, wherein the clamp-type sensor is electrically connected to or is electrically connectable to an electromagnetic or visual navigation system, preferably the cylindrical pin has a diameter of at least 3 mm and a length of at least 5 mm.
Clause 78. The balloon dilation system according to any one of the preceding clauses, wherein the inflation arrangement comprises a permanently mounted electromagnetic sensor or emitter.
Clause 79. The balloon dilation system according to any one of the preceding clauses, wherein the balloon dilation system comprises a filling port for filling liquid into the syringe and into the balloon catheter while the syringe and the balloon catheter are fluidically connected.
Clause 80. The balloon dilation system according to any one of the preceding clauses, wherein the rigid connection portion comprises a glued connection between the proximal shaft portion and the syringe, preferably said lumen port is glued directly to said syringe port.
Clause 81. The balloon dilation system according to any one of the preceding clauses, wherein the syringe comprises a spring element, wherein the spring element is located between the thruster and the plunger.
Clause 82. The balloon dilation system according to any one of the preceding clauses, wherein the balloon dilation system comprises a pressure indicator in fluid connection with the balloon, wherein the pressure indicator is adapted for visually, audibly and/or tactilely indicating if the balloon pressure is within a selected pressure range, preferably the pressure indicator comprises at least one of a pressure gauge and/or a visible spring loaded piston).
Clause 83. The balloon dilation system according to any one of the preceding clauses, wherein the balloon dilation system comprises a pressure regulator in fluid connection with the balloon, wherein the pressure regulator is adapted for preventing a pressure in the balloon from exceeding a threshold pressure, preferably the pressure regulator comprises at least one of a pressure relief valve, a pressure shut off valve and/or a plunger comprising a force limiting mechanism.
Clause 84. The balloon dilation system according to any one of the preceding clauses, wherein the balloon dilation system is suitable for balloon dilation of a Eustachian Tube of an individual.
Clause 85. The balloon dilation system according to any one of the preceding clauses, wherein the balloon dilation system is suitable for balloon dilation of at least one Sinus passageway of an individual.
Clause 86. The balloon dilation system according to any one of the preceding clauses, wherein the thruster is operably coupled to drive the plunger in a distal direction for inflation of the balloon.
Clause 87. A balloon dilation kit of parts comprising
   a balloon catheter comprising;
      a catheter shaft having a shaft length, a shaft axis, a proximal shaft portion, a distal shaft portion and a shaft lumen,
      an inflatable balloon located at the distal shaft portion, the balloon having a proximal balloon end and a distal balloon end and the balloon being in fluid connection with the shaft lumen, and
      a lumen port at the proximal shaft portion in fluid connection with the shaft lumen,
   an inflation arrangement comprising;
      a syringe comprising a barrel having a barrel volume, a syringe axis, and a plunger with a plunger rod and a plunger head,
      a syringe port in fluid connection with the barrel volume,
      a grasping portion engaged with, connected to or forming part of one of the plunger and the barrel,
      a thruster engaged with, connected to or forming part of another one of the plunger and the barrel,
   wherein the barrel volume is in fluid connection with the shaft lumen,
   wherein the balloon catheter and the inflation arrangement are adapted for being directly coupled to or integrated with each other to form a rigid connection portion. wherein the rigid connection portion is in a fully rigid connection portion wherein all motions of the entire inflation arrangement are translated to the balloon catheter
   wherein the grasping portion optionally is located with a first distance d1 to the proximal balloon end and the thruster is located with a second distance d2 to the proximal balloon end and wherein the second distance d2 is larger than the first distance d1; and/or
   wherein the inflation arrangement optionally has a distal end defined by the syringe port and wherein the grasping portion is located distally to the thruster, and
Clause 88. The balloon dilation kit of clause 87, wherein the kit comprises at least two balloon catheters having a distal shaft portions that differs from each other, such as distal shaft portions that differs from each, preferably a first of the at least two balloon catheter has a fixed length section pre-bent to have a first curved shape and or angle relative to the proximal shaft portion and a second of the at least two balloon catheter has a fixed length section pre-bent to have a second curved shape and or angle relative to the proximal shaft portion and a second of, wherein the first curved shape and or angle relative to the proximal shaft portion differs from the second curved shape and or angle relative to the proximal shaft portion.
Clause 89. The balloon dilation kit of clause 87 or clause 88, wherein the proximal shaft portion of the balloon catheter comprises a visually marked area adapted for clamping on a clamp-type sensor and wherein the kit comprises a clamp sensor adapted for being clamped to the visually marked area of the proximal shaft portion, preferably at least a length section of the proximal shaft portion comprises an externally located steel tube comprising the visually marked area.
Clause 90. The balloon dilation kit of any one of clauses 87-89, wherein the inflation arrangement comprises a protruding cylindrical pin and wherein the kit comprises a clamp sensor adapted for being clamped to the protruding cylindrical pin comprises a clamp-type sensor clamped on to the protruding cylindrical pin.
Clause 91. The balloon dilation kit of any one of clauses 87-90, wherein the kit comprises an electromagnetic sensor configured for being inserted into a working channel of the balloon catheter.
Clause 92. The balloon dilation kit of any one of clauses 87-91, wherein the kit comprises a light fiber configured for being inserted into a working channel of the balloon catheter.
Clause 93. The balloon dilation kit of any one of clauses 87-92, wherein all parts of the kit are packed in one packaging and sterilized together.
Clause 94. A first method for balloon dilation of an anatomic passageway in the head of a person/individual, the method using the balloon dilation system of any one of clauses 1-86, wherein the method comprising:
   a) Optionally shaping the distal end of the balloon catheter
   b) with one hand grasping the grasping portion, moving the catheter shaft forward into the nostril of a person
   c) with the same one hand grasping the grasping portion, moving the distal shaft portion transverse to the insertion direction to push the balloon into a Sinus passageway or into the Eustachian Tube
   d) with the thumb of the same one hand, pushing the thruster in a distal direction towards the balloon and relative to the grasping portion for inflating the balloon to thereby dilate the passageway,
   e) optionally deflating the balloon by retracting or releasing the thruster,
   f) removing the balloon catheter from the passageway and from the natural opening.
Clause 95. A second method for balloon dilation of an anatomic passageway in the head of a person, the method using the balloon dilation system of any one of clauses 1-86, wherein the method comprising:
   a) Clamping a clamp-type navigation sensor onto a part of the Balloon Dilation System
   b) Calibrating the navigation sensor to track the most distal tip of the balloon catheter
   c) with one hand grasping the grasping portion, moving the catheter shaft forward into the nostril of a person
   d) with the same one hand grasping the grasping portion, moving the distal shaft portion transverse to the insertion direction to push the balloon into a Sinus passageway or into the Eustachian Tube
   e) confirm placement of the balloon in the passageway by means of the navigation sensor and a navigation system.
   f) with the thumb of the same one hand, pushing the thruster in a distal direction towards the balloon and relative to the grasping portion for inflating the balloon to thereby dilate the passageway,
   g) optionally deflating the balloon by retracting or releasing the thruster,
   h) removing the balloon catheter from the passageway and from the natural opening.
Clause 96. A balloon dilation system for balloon dilation of an anatomic passageway, the balloon dilation system is optionally in accordance with any one if clauses 1-86, the balloon dilation system comprising:
   a balloon catheter and an inflation arrangement,
   wherein the balloon catheter comprising
      a catheter shaft having a shaft length, a shaft axis, a proximal shaft portion, a distal shaft portion and a shaft lumen,
      an inflatable balloon located at the distal shaft portion, the inflatable balloon having a proximal balloon end and a distal balloon end and the inflatable balloon being in fluid connection with the shaft lumen, and
      a lumen port at the proximal shaft portion in fluid connection with the shaft lumen, and
   wherein the inflation arrangement comprising
      a syringe comprising a barrel having a barrel volume,
      a syringe port in fluid connection with the barrel volume,
      a grasping portion engaged with, connected to or forming part of the barrel,
      a plunger with a plunger rod and a plunger head,
      a thruster slidably coupled to the plunger rod,
      a spring positioned between part of the plunger and part of the thruster,
      wherein an axial force applied to the thruster in a distal direction is transferred through the spring to the plunger and wherein the thruster can be moved distally relative to the plunger by compression of the spring.

## Claims

1. A balloon dilation system suitable for balloon dilation of an anatomic passageway, the balloon dilation system comprising:
a balloon catheter comprising;
a catheter shaft having a shaft length, a shaft axis, a proximal shaft portion, a distal shaft portion and a shaft lumen,
an inflatable balloon located at the distal shaft portion, the inflatable balloon having a proximal balloon end and a distal balloon end and the inflatable balloon being in fluid connection with the shaft lumen, and
a lumen port at the proximal shaft portion in fluid connection with the shaft lumen,
an inflation arrangement comprising;
a syringe comprising a barrel having a barrel volume, a syringe axis, and a plunger with a plunger rod and a plunger head,
a syringe port in fluid connection with the barrel volume,
a grasping portion engaged with, connected to or forming part of one of the plunger and the barrel,
a thruster engaged with, connected to or forming part of another one of the plunger and the barrel,
wherein the barrel volume is in fluid connection with the shaft lumen,
wherein the balloon dilation system comprises a rigid connection portion,
wherein the balloon catheter and the inflation arrangement are coupled to or are integrated with each other in said rigid connection portion and
wherein the rigid connection portion is in a fully rigid connection portion wherein all motions of the entire inflation arrangement are translated to the balloon catheter.

2. The balloon dilation system according to claim 1, wherein the proximal shaft portion extends at least 50 mm from the rigid connection portion and wherein the proximal shaft portion determined in a length section L extending from the rigid connection portion to 50 mm or more from the rigid connection portion has a flexural stiffness of at least 1000 Nmm², preferably the length section L extending from the rigid connection portion to 50 mm or more from the rigid connection portion has a flexural stiffness of at least 5000 Nmm², such as at least 10000 Nmm², such as at least 25000 Nmm², such as at least 50000 Nmm², such as at least 75000 Nmm², such as at least 80000 Nmm², preferably determined by the three-point-bending test method and/or by the single sided clamp method.

3. The balloon dilation system according to any one of the preceding claims, wherein the proximal shaft portion has a force-to-deflection ratio k determined using the single sided clamp method, wherein the clamp location is adjacent a proximal shaft end of the proximal shaft portion and the force applying location is adjacent the proximal shaft portion distal end and wherein the force-to-deflection ratio k is at least 0.1 N/mm, such as at least 0.2 N/mm, such as, at least 0.3 N/mm, such as, at least 0.4 N/mm, such as at least 0.5 N/mm, such as at least 0.6 N/mm, such as at least 0.7 N/mm, such as at least 0.8 N/mm.

4. The balloon dilation system according to any one of the preceding claims, wherein the grasping portion is located with a first distance d1 to the proximal balloon end and the thruster is located with a second distance d2 to the proximal balloon end and wherein the second distance d2 is larger than the first distance d1; and/or
wherein the inflation arrangement has a distal end defined by the syringe port and wherein the grasping portion is located distally to the thruster.

5. The balloon dilation system according to any one of the preceding claims, wherein the rigid connection portion comprises a coupling, such as a mechanical coupling, preferably said mechanical coupling comprises said fluid coupling, optionally said mechanical coupling is a separable mechanically coupling.

6. The balloon dilation system according to any one of the preceding claims, wherein the rigid connection portion comprises a glued coupling or a partly mechanical-partly glued coupling, wherein the rigid connection portion comprises a bonding material, such as an UV cross-linked polymer.

7. The balloon dilation system according to any one of the preceding claims 1-13, wherein the rigid connection portion comprises an integration portion integrating said balloon catheter and said inflation arrangement with each other, preferably to provide that the balloon catheter and the inflation arrangement form one unified structure.

8. The balloon dilation system according to claim 16, wherein the integration portion comprises at least an integration between said syringe port and said lumen port, preferably to ensure the fluid connection between the barrel volume and the shaft lumen.

9. The balloon dilation system according to any one of the preceding claims, wherein the rigid connection portion is sufficiently strong to withstand a bending moment from the catheter shaft of at least 50 N/mm, such as a bending moment of at least 60 N/mm, such as a bending moment of at least 75 N/mm or higher.

10. The balloon dilation system according to any one of the preceding claims, wherein the proximal shaft portion extends from the rigid connection portion and to a proximal shaft portion distal end located at least 70 mm from the rigid connection portion.

11. The balloon dilation system according to any one of the preceding claims, wherein the rigid connection portion directly interconnects the barrel and the catheter shaft, preferably the rigid connection portion comprises a mechanical coupling and wherein the mechanical coupling is establishing a rigid coupling directly between the barrel and the catheter shaft.

12. The balloon dilation system according to any one of the preceding claims, wherein the balloon dilation system comprises a filling port for filling liquid into the syringe and into the balloon catheter while the syringe and the balloon catheter are fluidically connected.

13. The balloon dilation system according to any one of the preceding claims, wherein the rigid connection portion comprises a glued connection between the proximal shaft portion and the syringe, preferably said lumen port is glued directly to said syringe port.

14. The balloon dilation system according to any one of the preceding claims, wherein the thruster is operably coupled to drive the plunger in a distal direction for inflation of the balloon.

15. The balloon dilation system according to any one of the preceding claims wherein the syringe comprises a spring element, wherein the spring element is located between the thruster and the plunger.
